# EUROPEAN PATENT SPECIFICATION

(11) **EP 3 352 777 B1**
(45) Date of publication and mention of the grant of the patent: **15.09.2021**
(21) Application number: 16777596.4
(22) Date of filing: 23.09.2016
(51) Int. Cl.: A61K 38/17, A61P 25/16, A61P 25/28

(54) **ENGRAILED FOR USE IN THE TREATMENT OF DNA-DAMAGE IN A PATIENT SUFFERING FROM PARKINSON DISEASE**
ENGRAILED ZUR VERWENDUNG IN DER BEHANDLUNG VON DNA-SCHÄDEN IN EINEM PATIENTEN DER AN PARKINSON LEIDET
ENGRAILED POUR L'UTILISATION DANS LE TRAITEMENT DES DÉGÂTS D'ADN CHEZ UN PATIENT SOUFFRANT DU PARKINSON

(30) Priority: 23.09.2015 EP 15306482
(43) Date of publication of application: 01.08.2018
(60) Divisional application: 21189549.5
(73) Proprietor: Centre National de la Recherche Scientifique, 75016 Paris (FR); Institut National de la Santé et de la Recherche Médicale (INSERM), 75013 Paris (FR); College de France, 75231 Paris Cedex 05 (FR); Sorbonne Université, 75006 Paris (FR)
(72) Inventor: PROCHIANTZ, Alain, 75006 Paris (FR)
(74) Representative: Gevers & Orès
(86) International application number: PCT/EP2016/072675
(87) International publication number: WO 2017/071889

(56) References cited:
- WO-A1-2013/128239
- WO-A2-2004/104030
- FR-A1- 2 662 698
- DANIEL ALVAREZ-FISCHER ET AL: "Engrailed protects mouse midbrain dopaminergic neurons against mitochondrial complex I insults", NATURE NEUROSCIENCE, vol. 14, no. 10, 1 January 2011 (2011-01-01), pages 1260-1266, XP055043540, ISSN: 1097-6256, DOI: 10.1038/nn.2916 cited in the application
- TORERO IBAD R ET AL: "Otx2 Promotes the Survival of Damaged Adult Retinal Ganglion Cells and Protects against Excitotoxic Loss of Visual Acuity In Vivo", JOURNAL OF NEUROSCIENCE, SOCIETY FOR NEUROSCIENCE, US, vol. 31, no. 14, 6 April 2011 (2011-04-06) , pages 5495-5503, XP002699685, ISSN: 0270-6474, DOI: 10.1523/JNEUROSCI.0187-11.2011
- J. SPATAZZA ET AL: "Homeoprotein Signaling in Development, Health, and Disease: A Shaking of Dogmas Offers Challenges and Promises from Bench to Bed", PHARMACOLOGICAL REVIEWS, vol. 65, no. 1, 8 January 2013 (2013-01-08), pages 90-104, XP055173064, DOI: 10.1124/pr.112.006577
- A. PROCHIANTZ ET AL: "Postnatal signalling with homeoprotein transcription factors", JOURNAL OF BIOLOGICAL CHEMISTRY, vol. 282, no. 12, 18 August 2014 (2014-08-18), pages 8994-20130518, XP055173065, ISSN: 0021-9258, DOI: 10.1074/jbc.M609246200
- HOCINE REKAIK ET AL: "Engrailed Homeoprotein Protects Mesencephalic Dopaminergic Neurons from Oxidative Stress", CELL REPORTS, vol. 13, no. 2, 24 September 2015 (2015-09-24), pages 242-250, XP055273273, US ISSN: 2211-1247, DOI: 10.1016/j.celrep.2015.08.076
- MATIAS ALVAREZ-SAAVEDRA ET AL: "Coordinated epigenetic regulation of Engrailed-1 by the chromatin remodelers Smarca1 and Smarca5 mediates cerebellar morphogenesis", EPIGENETICS & CHROMATIN, BIOMED CENTRAL LTD, LONDON, UK, vol. 6, no. Suppl 1, 8 April 2013 (2013-04-08), page P105, XP021147529, ISSN: 1756-8935, DOI: 10.1186/1756-8935-6-S1-P105
- FRANÇOIS-XAVIER BLAUDIN DE THÉ ET AL: "Neuroprotective Transcription Factors in Animal Models of Parkinson Disease", NEURAL PLASTICITY, vol. 20, no. 4, 31 December 2015 (2015-12-31), pages 1275-11, XP055273348, ISSN: 2090-5904, DOI: 10.1016/j.neuron.2015.01.019
- BERNSTEIN ALISON I ET AL: "6-OHDA generated ROS induces DNA damage and p53- and PUMA-dependent cell death", MOLECULAR NEURODEGENERATION, BIOMED CENTRAL LTD, LO, vol. 6, no. 1, 6 January 2011 (2011-01-06) , page 2, XP021090446, ISSN: 1750-1326, DOI: 10.1186/1750-1326-6-2

### SEQUENCE LISTING

<110> Centre National de la Recherche Scientifique Institut National de la Recherche Medicale College de France
<120> Treatment of DNA-damage and age-related diseases or disorders
<130> F0644/381WO ELMA/safl
<150> EP15306482.9
   <151> 2015-09-23
<160> 50
<170> PatentIn version 3.5
<210> 1
   <211> 60
   <212> PRT
   <213> artificial sequence
<220>
   <223> synthetic polypeptide
<400> 1
   Arg Arg Arg Lys Arg Thr Ala Tyr Thr Arg Tyr Gln Leu Leu Glu Leu
   1               5                   10                  15
   Glu Lys Glu Phe Leu Phe Asn Arg Tyr Leu Thr Arg Arg Arg Arg Ile
               20                  25                  30
   Glu Leu Ala His Ser Leu Asn Leu Thr Glu Arg His Ile Lys Ile Trp
           35                  40                  45
   Phe Gln Asn Arg Arg Met Lys Trp Lys Lys Glu Asn
       50                  55                  60
<210> 2
   <211> 401
   <212> PRT
   <213> Mus musculus
<400> 2
   Met Glu Glu Gln Gln Pro Glu Pro Lys Ser Gln Arg Asp Ser Gly Leu
   1               5                   10                  15
   Gly Ala Val Ala Ala Ala Ala Pro Ser Gly Leu Ser Leu Ser Leu Ser
               20                  25                  30
   Pro Gly Ala Ser Gly Ser Ser Gly Ser Asp Gly Asp Ser Val Pro Val
           35                  40                  45
   Ser Pro Gln Pro Ala Pro Pro Ser Pro Pro Ala Ala Pro Cys Leu Pro
       50                  55                  60
   Pro Leu Ala His His Pro His Leu Pro Pro His Pro Pro Pro Pro Pro
   65                  70                  75                  80
   Pro Pro Pro Pro Pro Pro Pro Gln His Leu Ala Ala Pro Ala His Gln
                   85                  90                  95
   Pro Gln Pro Ala Ala Gln Leu His Arg Thr Thr Asn Phe Phe Ile Asp
               100                 105                 110
   Asn Ile Leu Arg Pro Asp Phe Gly Cys Lys Lys Glu Gln Pro Leu Pro
           115                 120                 125
   Gln Leu Leu Val Ala Ser Ala Ala Ala Gly Gly Gly Ala Ala Ala Gly
       130                 135                 140
   Gly Gly Ser Arg Val Glu Arg Asp Arg Gly Gln Thr Gly Ala Gly Arg
   145                 150                 155                 160
   Asp Pro Val His Ser Leu Gly Thr Arg Ala Ser Gly Ala Ala Ser Leu
                   165                 170                 175
   Leu Cys Ala Pro Asp Ala Asn Cys Gly Pro Pro Asp Gly Ser Gln Pro
               180                 185                 190
   Ala Thr Ala Val Gly Ala Gly Ala Ser Lys Ala Gly Asn Pro Ala Ala
           195                 200                 205
   Ala Ala Ala Ala Ala Ala Ala Ala Ala Ala Ala Ala Val Ala Ala Ala
       210                 215                 220
   Ala Ala Ala Ala Ser Lys Pro Ser Asp Ser Gly Gly Gly Ser Gly Gly
   225                 230                 235                 240
   Asn Ala Gly Ser Pro Gly Ala Gln Gly Ala Lys Phe Pro Glu His Asn
                   245                 250                 255
   Pro Ala Ile Leu Leu Met Gly Ser Ala Asn Gly Gly Pro Val Val Lys
               260                 265                 270
   Thr Asp Ser Gln Gln Pro Leu Val Trp Pro Ala Trp Val Tyr Cys Thr
           275                 280                 285
   Arg Tyr Ser Asp Arg Pro Ser Ser Gly Pro Arg Thr Arg Lys Leu Lys
       290                 295                 300
   Lys Lys Lys Asn Glu Lys Glu Asp Lys Arg Pro Arg Thr Ala Phe Thr
   305 310 315 320
   Ala Glu Gln Leu Gln Arg Leu Lys Ala Glu Phe Gln Ala Asn Arg Tyr
   325 330 335
   Ile Thr Glu Gln Arg Arg Gln Thr Leu Ala Gln Glu Leu Ser Leu Asn
   340 345 350
   Glu Ser Gln Ile Lys Ile Trp Phe Gln Asn Lys Arg Ala Lys Ile Lys
   355 360 365
   Lys Ala Thr Gly Ile Lys Asn Gly Leu Ala Leu His Leu Met Ala Gln
   370 375 380
   Gly Leu Tyr Asn His Ser Thr Thr Thr Val Gln Asp Lys Asp Glu Ser
   385 390 395 400
   Glu
<210> 3
   <211> 348
   <212> PRT
   <213> Mus musculus
<400> 3
   Met Ser Ala Ala Phe Pro Pro Ser Leu Met Met Met Gln Arg Pro Leu
   1 5 10 15
   Gly Ser Ser Thr Ala Phe Ser Ile Asp Ser Leu Ile Gly Ser Pro Pro
   20 25 30
   Gln Pro Ser Pro Gly His Phe Val Tyr Thr Gly Tyr Pro Met Phe Met
   35 40 45
   Pro Tyr Arg Pro Val Val Leu Pro Pro Pro Pro Pro Pro Pro Pro Ala
   50 55 60
   Leu Pro Gln Ala Ala Leu Gln Pro Ala Leu Pro Pro Ala His Pro His
   65 70 75 80
   His Gln Ile Pro Ser Leu Pro Thr Gly Phe Cys Ser Ser Leu Ala Gln
   85 90 95
   Gly Met Ala Leu Thr Ser Thr Leu Met Ala Thr Leu Pro Gly Gly Phe
   100 105 110
   Ser Ala Ser Pro Gln His Gln Glu Ala Ala Ala Ala Arg Lys Phe Ala
           115                 120                 125
   Pro Gln Pro Leu Pro Gly Gly Gly Asn Phe Asp Lys Ala Glu Ala Leu
       130                 135                 140
   Gln Ala Asp Ala Glu Asp Gly Lys Ala Phe Leu Ala Lys Glu Gly Ser
   145                 150                 155                 160
   Leu Leu Ala Phe Ser Ala Ala Glu Ala Val Gln Ala Ser Leu Val Gly
                   165                 170                 175
   Ala Val Arg Gly Gln Gly Lys Asp Glu Ser Lys Val Glu Asp Asp Pro
               180                 185                 190
   Lys Gly Lys Glu Glu Ser Phe Ser Leu Glu Ser Asp Val Asp Tyr Ser
           195                 200                 205
   Ser Asp Asp Asn Leu Pro Gly Gln Thr Ala His Lys Glu Glu Asp Pro
       210                 215                 220
   Gly His Ala Leu Glu Glu Thr Pro Gln Ser Gly Gly Ala Ala Gly Ser
   225                 230                 235                 240
   Thr Thr Ser Thr Gly Lys Asn Arg Arg Arg Arg Thr Ala Phe Thr Ser
                   245                 250                 255
   Glu Gln Leu Leu Glu Leu Glu Lys Glu Phe His Cys Lys Lys Tyr Leu
               260                 265                 270
   Ser Leu Thr Glu Arg Ser Gln Ile Ala His Ala Leu Lys Leu Ser Glu
           275                 280                 285
   Val Gln Val Lys Ile Trp Phe Gln Asn Arg Arg Ala Lys Trp Lys Arg
       290                 295                 300
   Val Lys Ala Gly Asn Ala Asn Ser Lys Thr Gly Glu Pro Ser Arg Asn
   305                 310                 315                 320
   Pro Lys Ile Val Val Pro Ile Pro Val His Val Ser Arg Phe Ala Ile
                   325                 330                 335
   Arg Ser Gln His Gln Gln Leu Glu Gln Ala Arg Pro
               340                 345
<210> 4
   <211> 397
   <212> PRT
   <213> Mus musculus
<400> 4
   Met Glu Ile Val Gly Cys Arg Ala Glu Asn Asn Ser Cys Pro Phe Arg
   1               5                   10                  15
   Pro Pro Ala Met Leu Phe His Gly Ile Ser Gly Gly His Ile Gln Gly
               20                  25                  30
   Ile Met Glu Glu Met Glu Arg Arg Ser Lys Thr Glu Ala Arg Leu Thr
           35                  40                  45
   Lys Gly Thr Gln Leu Asn Gly Arg Asp Ala Gly Met Pro Pro Leu Ser
       50                  55                  60
   Pro Glu Lys Pro Ala Leu Cys Ala Gly Cys Gly Gly Lys Ile Ser Asp
   65                  70                  75                  80
   Arg Tyr Tyr Leu Leu Ala Val Asp Lys Gln Trp His Leu Arg Cys Leu
                   85                  90                  95
   Lys Cys Cys Glu Cys Lys Leu Ala Leu Glu Ser Glu Leu Thr Cys Phe
               100                 105                 110
   Ala Lys Asp Gly Ser Ile Tyr Cys Lys Glu Asp Tyr Tyr Arg Arg Phe
           115                 120                 125
   Ser Val Gln Arg Cys Ala Arg Cys His Leu Gly Ile Ser Ala Ser Glu
       130                 135                 140
   Met Val Met Arg Ala Arg Asp Ser Val Tyr His Leu Ser Cys Phe Thr
   145                 150                 155                 160
   Cys Ser Thr Cys Asn Lys Thr Leu Thr Thr Gly Asp His Phe Gly Met
                   165                 170                 175
   Lys Asp Ser Leu Val Tyr Cys Arg Ala His Phe Glu Thr Leu Leu Gln
               180                 185                 190
   Gly Glu Tyr Pro Pro Gln Leu Ser Tyr Thr Glu Leu Ala Ala Lys Ser
           195                 200                 205
   Gly Gly Leu Ala Leu Pro Tyr Phe Asn Gly Thr Gly Thr Val Gln Lys
       210                 215                 220
   Gly Arg Pro Arg Lys Arg Lys Ser Pro Ala Leu Gly Val Asp Ile Val
   225                 230                 235                 240
   Asn Tyr Asn Ser Gly Cys Asn Glu Asn Glu Ala Asp His Leu Asp Arg
   245 250 255
   Asp Gln Gln Pro Tyr Pro Pro Ser Gln Lys Thr Lys Arg Met Arg Thr
   260 265 270
   Ser Phe Lys His His Gln Leu Arg Thr Met Lys Ser Tyr Phe Ala Ile
   275 280 285
   Asn His Asn Pro Asp Ala Lys Asp Leu Lys Gln Leu Ala Gln Lys Thr
   290 295 300
   Gly Leu Thr Lys Arg Val Leu Gln Val Trp Phe Gln Asn Ala Arg Ala
   305 310 315 320
   Lys Phe Arg Arg Asn Leu Leu Arg Gln Glu Asn Gly Gly Val Asp Lys
   325 330 335
   Ala Asp Gly Thr Ser Leu Pro Ala Pro Pro Ser Ala Asp Ser Gly Ala
   340 345 350
   Leu Thr Pro Pro Gly Thr Ala Thr Thr Leu Thr Asp Leu Thr Asn Pro
   355 360 365
   Thr Val Thr Val Val Thr Thr Val Thr Ser Asn Met Asp Ser His Glu
   370 375 380
   Pro Gly Ser Pro Ser Gln Thr Thr Leu Thr Asn Leu Phe
   385 390 395
<210> 5
   <211> 20
   <212> DNA
   <213> artificial sequence
<220>
   <223> synthetic oligonucleotide
<400> 5
   tggctcctgg tacatcgaga 20
<210> 6
   <211> 20
   <212> DNA
   <213> artificial sequence
<220>
   <223> Synthetic oligonucleotide
<400> 6
   agcattggca accctgagaa 20
<210> 7
   <211> 17
   <212> DNA
   <213> artificial sequence
<220>
   <223> Synthetic oligonucleotide
<400> 7
   gggcctgctc aggctgt 17
<210> 8
   <211> 20
   <212> DNA
   <213> artificial sequence
<220>
   <223> Synthetic oligonucleotide
<400> 8
   gttctctgtg ggggatcctg 20
<210> 9
   <211> 20
   <212> DNA
   <213> artificial sequence
<220>
   <223> Synthetic oligonucleotide
<400> 9
   ctcgctgcat caggaagacc 20
<210> 10
   <211> 19
   <212> DNA
   <213> artificial sequence
<220>
   <223> Synthetic oligonucleotide
<400> 10
   taagaggagc aacccgtcg 19
<210> 11
   <211> 21
   <212> DNA
   <213> artificial sequence
<220>
   <223> Synthetic oligonucleotide
<400> 11
   gccagactat aagccctacc c 21
<210> 12
   <211> 20
   <212> DNA
   <213> artificial sequence
<220>
   <223> Synthetic oligonucleotide
<400> 12
   gctgcacagg gttacacttc 20
<210> 13
   <211> 20
   <212> DNA
   <213> artificial sequence
<220>
   <223> Synthetic oligonucleotide
<400> 13
   ccggtggtca agactgactc 20
<210> 14
   <211> 18
   <212> DNA
   <213> artificial sequence
<220>
   <223> Synthetic oligonucleotide
<400> 14
   ctggtgcgtg gaccagag 18
<210> 15
   <211> 20
   <212> DNA
   <213> artificial sequence
<220>
   <223> Synthetic oligonucleotide
<400> 15
   ccttcttcag gtcccaggtc 20
<210> 16
   <211> 20
   <212> DNA
   <213> artificial sequence
<220>
   <223> Synthetic oligonucleotide
<400> 16
   aactcagcct tgagcctctg 20
<210> 17
   <211> 19
   <212> DNA
   <213> artificial sequence
<220>
   <223> Synthetic oligonucleotide
<400> 17
   attcctcttc gccgccatt 19
<210> 18
   <211> 20
   <212> DNA
   <213> artificial sequence
<220>
   <223> Synthetic oligonucleotide
<400> 18
   gaggcatcga tggccacttt 20
<210> 19
   <211> 22
   <212> DNA
   <213> artificial sequence
<220>
   <223> Synthetic oligonucleotide
<400> 19
   tctctagagg aacgctgaga cc 22
<210> 20
   <211> 22
   <212> DNA
   <213> artificial sequence
<220>
   <223> Synthetic oligonucleotide
<400> 20
   gtagggtagc ctttgaggga tt 22
<210> 21
   <211> 20
   <212> DNA
   <213> artificial sequence
<220>
   <223> Synthetic oligonucleotide
<400> 21
   cccacctgtc agacccaagt 20
<210> 22
   <211> 23
   <212> DNA
   <213> artificial sequence
<220>
   <223> Synthetic oligonucleotide
<400> 22
   agtcatagac cagccctgta gca 23
<210> 23
   <211> 20
   <212> DNA
   <213> artificial sequence
<220>
   <223> Synthetic oligonucleotide
<400> 23
   ctgggaactg ccaaagcaac 20
<210> 24
   <211> 20
   <212> DNA
   <213> artificial sequence
<220>
   <223> Synthetic oligonucleotide
<400> 24
   cctccgttta cctttcgcca 20
<210> 25
   <211> 23
   <212> DNA
   <213> artificial sequence
<220>
   <223> Synthetic oligonucleotide
<400> 25
   aatcgacaaa tgggacctaa tga 23
<210> 26
   <211> 25
   <212> DNA
   <213> artificial sequence
<220>
   <223> Synthetic oligonucleotide
<400> 26
   gtaaagatcc tttcccaatc tgttg 25
<210> 27
   <211> 20
   <212> DNA
   <213> artificial sequence
<220>
   <223> Synthetic oligonucleotide
<400> 27
   ggtggaagac aaggaggaag 20
<210> 28
   <211> 19
   <212> DNA
   <213> artificial sequence
<220>
   <223> Synthetic oligonucleotide
<400> 28
   aggaggagaa aaagccgag 19
<210> 29
   <211> 18
   <212> DNA
   <213> artificial sequence
<220>
   <223> Synthetic oligonucleotide
<400> 29
   ggcccggaac atctcagg 18
<210> 30
   <211> 21
   <212> DNA
   <213> artificial sequence
<220>
   <223> Synthetic oligonucleotide
<400> 30
   aaatctgtca ggctggtctg c 21
<210> 31
   <211> 20
   <212> DNA
   <213> artificial sequence
<220>
   <223> Synthetic oligonucleotide
<400> 31
   ggcgtaaacg cttcgagatg 20
<210> 32
   <211> 21
   <212> DNA
   <213> artificial sequence
<220>
   <223> Synthetic oligonucleotide
<400> 32
   cttcaggtag ctggagtgag c 21
<210> 33
   <211> 20
   <212> DNA
   <213> artificial sequence
<220>
   <223> Synthetic oligonucleotide
<400> 33
   gaccccagct tgaagagtcc 20
<210> 34
   <211> 20
   <212> DNA
   <213> artificial sequence
<220>
   <223> Synthetic oligonucleotide
<400> 34
   ctgcccatcc agttcgagtt 20
<210> 35
   <211> 20
   <212> DNA
   <213> artificial sequence
<220>
   <223> Synthetic oligonucleotide
<400> 35
   ctatccggat gttcgcctcc 20
<210> 36
   <211> 20
   <212> DNA
   <213> artificial sequence
<220>
   <223> Synthetic oligonucleotide
<400> 36
   gaggtgacct atctgggggt 20
<210> 37
   <211> 20
   <212> DNA
   <213> artificial sequence
<220>
   <223> Synthetic oligonucleotide
<400> 37
   atagcagcag tgagtccagc 20
<210> 38
   <211> 23
   <212> DNA
   <213> artificial sequence
<220>
   <223> Synthetic oligonucleotide
<400> 38
   gctggctaat tttctgggtt tca 23
<210> 39
   <211> 20
   <212> DNA
   <213> artificial sequence
<220>
   <223> Synthetic oligonucleotide
<400> 39
   cacagcactt gcaggaaagg 20
<210> 40
   <211> 20
   <212> DNA
   <213> artificial sequence
<220>
   <223> Synthetic oligonucleotide
<400> 40
   cctgtctggg tgtgtcgatt 20
<210> 41
   <211> 22
   <212> DNA
   <213> artificial sequence
<220>
   <223> Synthetic oligonucleotide
<400> 41
   cgtgtaagac attcctatct cg 22
<210> 42
   <211> 21
   <212> DNA
   <213> artificial sequence
<220>
   <223> Synthetic oligonucleotide
<400> 42
   gcccgctggc agaacgagaa g 21
<210> 43
   <211> 20
   <212> DNA
   <213> artificial sequence
<220>
   <223> Synthetic oligonucleotide
<400> 43
   tactgcttag tggccagcct 20
<210> 44
   <211> 20
   <212> DNA
   <213> artificial sequence
<220>
   <223> Synthetic oligonucleotide
<400> 44
   aggcatttgc ttgcacacac 20
<210> 45
   <211> 22
   <212> DNA
   <213> artificial sequence
<220>
   <223> Synthetic oligonucleotide
<400> 45
   gtactggaca gtcctcacac ca 22
<210> 46
   <211> 22
   <212> DNA
   <213> artificial sequence
<220>
   <223> Synthetic oligonucleotide
<400> 46
   ggtggtaccc tatgcattta gc 22
<210> 47
   <211> 20
   <212> DNA
   <213> artificial sequence
<220>
   <223> Synthetic oligonucleotide
<400> 47
   agaggtcaag aagggcaagc 20
<210> 48
   <211> 20
   <212> DNA
   <213> artificial sequence
<220>
   <223> Synthetic oligonucleotide
<400> 48
   tgccactcaa cgctctttct 20
<210> 49
   <211> 20
   <212> DNA
   <213> artificial sequence
<220>
   <223> Synthetic oligonucleotide
<400> 49
   gcgtggccag tgtaaaagca 20
<210> 50
   <211> 21
   <212> DNA
   <213> artificial sequence
<220>
   <223> Synthetic oligonucleotide
<400> 50
   cgcattcttc acagatggtg t 21

## Description

The present invention relates to the Engrailed protein or a composition comprising the Engrailed protein for use in the prevention and/or treatment of DNA-damage in a patient suffering from Parkinson disease, wherein said Engrailed protein is injected once into the substantia nigra pars compacta (SNpc) region.

### BACKGROUND OF THE INVENTION

Study of aging processes is important, in part because many diseases or conditions become more prevalent among aged people, for example, cancers, Alzheimer's disease, Parkinson's disease, stroke, glaucoma, cardiovascular disease, just to name a few, among which many still lack effective preventive or treatment methods. Although abundant literature has contributed to the understanding of aging processes, full understanding of the processes remains a major challenge. Given the increasing population of aged people around the world and the rising health care burden and cost associated therewith, studies of the aging processes leading to effective discovery of anti-aging agents for the prevention or treatment of age-related diseases or disorders are becoming increasingly important.

Cellular DNA is under constant challenge by exogenous and endogenous genotoxic stresses (e.g. oxidative stress, toxic byproducts, reduced mitochondrial function) that modify DNA through base modification or mis-incorporation resulting in DNA damage. DNA damage is physical abnormality in the DNA structure such as single and double strand breaks. DNA damage can be recognized by specific enzymes, and thus can be correctly repaired through the DNA Damage Response (DDR). The loss of this natural DNA repair capacity results in genetic instability that may lead to cellular dysfunction and cell death. It has been further shown that chromatin remodeling and DDR pathways are interconnected as DNA damage induces chromatin changes, themselves necessary to give access to the DNA repair machinery (Soria et al., 2012, Mol Cell 46, 722-734).

Accumulation of DNA damage has been linked to the process of aging and to the onset of age-related diseases including neurodegenerative disorders, such as ataxias, Alzheimer's, amyotrophic lateral sclerosis, Huntington's and Parkinson's diseases (Canugovi et al., 2013, 12, 578-587; Madabhushi et al., 2014, Neuron 83, 266-282).

Additionally, in neurodegenerative disorders, such as ischemia and Alzheimer's disease, neurons manifest aberrant cell cycle activity, expressing cell cycle markers such as Cyclin-A, and undergo a limited extent of DNA remodeling. This behavior is remarkable considering that in humans neurons terminally differentiate during development and remain quiescent for decades prior to the onset of these events. While the underlying mechanisms are poorly understood, multiple lines of evidence suggest that these activities play an early and contributory role in neuronal death (Andorfer et al., 2005, J. Neurosci., 1,25, 5446-54).

It has further been shown that said DNA damage and re-entry into the cell cycle may constitute a common pathway in apoptosis in neurological diseases (Kim and Tsai, 2009, Ann N Y ACAD Sci., 1170, 674-9 or Pizarro et al., 2009, Free Radic. Res., 43, 985-994).

Therefore, there is a strong need to develop new therapeutic agents that promote DNA repair, that are able to prevent and/or treat DNA damage and/or cellular aging related disorders and/or regulate the cell cycle.

Homeoproteins, or homeodomain proteins, are transcription factors that play a major role in cell migration and differentiation processes involved in morphogenesis. They are characterized by the presence of a sequence of 60 amino acids, the homeodomain, which is a DNA binding domain with a helix/turn/helix structure. It has been shown that the isolated domain of Antennapedia protein of Drosophila crosses the membrane of neurons in culture, accumulates in the nucleus and promotes neurite growth (EP0485578). The homeodomain is highly conserved and confers the internalization property to a large number of homeoproteins (Spatazza et al., 2013, Pharmacol. Rev. 65, 90-104).

Engrailed proteins (Engrailed-1 and Engrailed-2) are homeoproteins with similar biological activity that are designated collectively hereafter with the general term Engrailed (EN for the human or En1/2 for mice). In neonates and in adults, Engrailed is expressed in cerebellar granule cells and in mesencephalic dopaminergic (DA) nuclei, including the substantia nigra pars compacta (SNpc that degenerates in Parkinson's disease), and the Ventral Tegmental Area (VTA). En1/2 plays important roles in the development of the midbrain and, in the midbrain, of the mesencephalic dopaminergic (mDA) neurons (Joyner, 1996, Trends Genet 12, 15-20). En1/2 also plays redundant roles in the survival of adult mDA neurons that are located in the SNpc and the VTA in the ventral part of the mesencephalon (Alberi et al., 2004, Development 131, 3229-3236), and therefore it was proposed for preventing or treating the loss of DA neurons in Parkinson disease. In WO 2013/128239, it was reported that local administration of En1/2 by infusion in the midbrain increases DA synthesis by DA neurons and associated motor activity. Prochiantz et al. (2011, FEBS Letters, 278, 52 (Abstract, Brunet et al., Nature 438 : 94-98, 2005 and Alvarez-Fisher et al., Nature Neurosci 14 : 1260-1266, 2011) have reported that Engrailed is not only a transcription factor but also a translation regulator that enhances the translation of mitochondrial mRNAs transcribed in the nucleus and it was shown that Engrailed transduction up-regulates the translation of Ndufs1 and Ndufs3, two proteins of mitochondrial complex I and increases ATP synthesis (see also Alvarez-Fischer et al., 2011, Nature Neuroscience, 14, 1260-1266, Stettler *et al.* 2012). Alternatively, in WO 2007/099227, it was shown that systemic administration of En1/2 to mice induces an increase in DA turnover in the striatum, reflected by an increase in the production of the DA metabolite 3,4-dihydroxyphenylacetic acid (DOPAC) without modification of dopamine levels. In Alvarez-Fischer *et al.,* 2011, it was shown that Engrailed protects mDA neurons in three mouse models of Parkinson disease.

Otx2 (orthodenticle homolog 2) is another homeoprotein containing a bicoid-type homeodomain (Simeone et al., 1993, EMBO J 12, 2735-2747). It belongs to the Otx protein family, which plays fundamental roles during brain embryogenesis (Acampora *et al.,* 1995; Simeone *et al.,* 2002). It has also been shown that Otx2 is involved in the formation of retina and in adult retinal ganglion cell survival and physiology (Bernard *et al.,* 2013; Torero Ibad et *al.,* 2011). It was thus proposed to use homeoproteins of the bicoid family, in particular of the Otx family, such as Otx2, for improving survival of adult retinal ganglionic neurons (RGCs) in culture *in vitro,* and also for preventing or treating, *in vivo,* RGC degeneration (WO 2009/106767).

The Inventors have now shown that homeoproteins are able to protect cells from DNA damage and chromatin remodeling, to decrease the number of DNA strand breaks, to restore all nuclear and nucleolar heterochromatin marks, to regulate the cell cycle and to protect cell against aging-like excitotoxicity, including apoptosis In particular, they showed that Engrailed, when administered into the SNpc region, is able to repair the damages leading to cell death provoked by a strong oxidative stress. In particular, they activate anti-apoptotic pathways and promote long-term survival by restoring heterochromatin marks, including nucleolus integrity, and by abolishing double-strands breaks in a model of acute oxidative stress. These results allow to propose to use homeoproteins, and in particular Engrailed, in pathologies wherein DNA damage occurs, such as in Parkinson disease.

According to the present Invention, the term "DNA damage" covers collectively DNA damage, DNA strand breaks (single and double strand), and nuclear and nucleolar heterochromatin disruption.

The invention is as defined in claims 1 to 4.

The invention relates to the Engrailed protein for use in the treatment and/or prevention of DNA-damage in a patient suffering from Parkinson disease, wherein said Engrailed protein is injected once into the substantia nigra pars compacta (SNpc) region.

In another aspect, the invention relates to a composition comprising Engrailed protein for use in the treatment and/or prevention of DNA-damage in a patient suffering from Parkinson disease, wherein said composition is injected once into the substantia nigra pars compacta (SNpc) region.

In an embodiment, the invention relates to a composition for use as defined above, wherein said composition comprises another pharmaceutical drug selected in the group of growth factor, complex sugar and sirtuin.

In an embodiment, the invention relates to a composition for use as defined above, wherein said complex sugar is polysialic acid.

The present disclosure relates to - but not claims - the use of at least one homeoprotein, or peptide derivatives thereof, for the treatment or prevention of DNA damage and/or cellular aging. The disclosure further concerns - but not claims - the use of at least one homeoprotein, or peptide derivatives thereof, for the treatment or prevention of conditions associated with DNA damage and/or cellular aging, and more particularly for the prevention and/or treatment of DNA-damage related diseases or disorders and age-related diseases or disorders.

The present disclosure relates to- but not claims - the use of a recombinant vector encoding said proteins or peptides for the treatment or prevention of DNA damage and/or cellular aging and for the treatment or prevention of conditions associated with DNA damage and/or cellular aging, and more particularly for the prevention and/or treatment of DNA-damage related diseases or disorders and age-related diseases or disorders.

As used herein, the terms "homeoprotein " and " homeodomain proteins " are synonymous and refer to gene products, proteins and polypeptides commonly known under these designations in the art. The terms encompass such gene products, proteins and polypeptides of any organism where found, and particularly of animals, preferably vertebrates, more preferably mammals, including non-human mammals, and even more preferably of humans. More particularly, "homeoprotein" relates to a protein comprising a homeodomain. Homeoproteins include natural homeoproteins and recombinant homeoproteins or synthetic homeoproteins.

The present invention encompasses native Engrailed protein, including allelic variants thereof. The Engrailed protein of the present invention comprises the full-length homeodomain.

Suitable variants of Engrailed protein for use in the present invention are biologically active and retain at least one of the activities described herein in connection with the corresponding native homeoprotein. Preferably, the effect on DNA damage and/or chromatin alteration is preserved, although a given function of the native Engrailed protein may be positively or negatively affected to some degree, e.g. with variants exhibiting reduced biological activity.

The Engrailed protein for use according to the invention can be prepared by the conventional techniques known to those skilled in the art, in particular by expression of a recombinant DNA in a suitable cell system (eukaryotic or prokaryotic) or by solid-phase or liquid-phase synthesis. More specifically, the Engrailed protein is usually produced from a DNA polynucleotide comprising a sequence coding for the protein, obtained by any means known to those skilled in the art, including amplification of a nucleic sequence by PCR or RT-PCR, screening genomic DNA libraries by hybridization with a homologous probe, or else total or partial chemical synthesis. The recombinant vectors are constructed and introduced into host cells by conventional recombinant DNA and genetic engineering techniques, which are known in the art. The DNA polynucleotide is cloned into a eukaryotic or prokaryotic expression vector and the protein produced in the cells modified with the recombinant vector is purified by any suitable means, in particular by affinity chromatography. The peptide is usually solid-phase synthesized, according to the Fmoc technique, originally described by Merrifield et al. (J. Am. Chem. Soc., 1964, 85: 2149-2154) and purified by reverse-phase high performance liquid chromatography.

According to more preferred embodiment, the terms designate proteins and polypeptides comprising the following amino acid sequence (homeodomain) RRRKRTA-YTRYQLLE-LEKEFLF-NRYLTRRRRIELAHSL-NLTERHIKIWFQN-RRMK-WKKEN [typical insertion sites are noted with dashes] (SEQ ID NO:1) (see http://homeobox.biosci.ki.se/).

Examples of homeoproteins which can be used include all homeoproteins expressed in different subsets of adult neurons such as: Engrailed proteins (Engrailed-1 or Engrailed-2), and - not claimed but for illustration purpose only - homeoproteins from the bicoïd family, in particular the Otx sub-family like Otx2, proteins from the paired family (Pax), the Lhx family, for example Lhx9, and Gbx2.

Homeoprotein gene sequences and deduced amino acid sequences are well known in the art and available in the sequence databases.

Exemplary En1/2 protein or polypeptide includes without limitation mouse En1/2 protein or polypeptide having primary amino acid sequence (SEQ ID NO: 2) :
musEn1: NP_034263 (401aa)

Exemplary Gbx2 protein or polypeptide includes without limitation mouse Gbx2 protein or polypeptide having primary amino acid sequence (SEQ ID NO: 3) - not claimed :
musGbx2: NP_034392 (longest variant, 348aa)

Exemplary Lhx9 protein or polypeptide includes without limitation mouse Lhx9 protein or polypeptide having primary amino acid sequence (SEQ ID NO: 4) - not claimed :
musLhx9: NP_001036042 (longest variant, 397aa)

Exemplary En1 protein or polypeptide includes without limitation human EN1 protein or polypeptide having primary amino acid sequence as annotated under Genbank accession number AAA 53502.2 or NCBI NP_001417.3.

Exemplary En2 protein or polypeptide includes without limitation human EN2 protein or polypeptide having primary amino acid sequence as annotated under Genbank accession number AAA 53504.2 or NCBI NP_001418.2.

Exemplary Gbx2 protein or polypeptide - not claimed - includes without limitation human Gbx2 protein or polypeptide having primary amino acid sequence as annotated under Genbank accession number EAW71084.1.

Exemplary Lhx9 protein or polypeptide - not claimed - includes without limitation human Lhx9 protein or polypeptide having primary amino acid sequence as annotated under Genbank accession number NP_001014434.1 or_ NP_064589.2.

According to the invention, the homeoprotein is Engrailed protein (Engrailed-1 or Engrailed-2). The fused domain may be from Engrailed protein. The Engrailed repressor domain may be the domain situated from positions 1 to 298 of Engrailed-1 amino acid sequence or a subdomain thereof such as the eh1 domain (positions 46 to 67 of Engrailed-2 amino acid sequence).

According to another preferred embodiment of the invention, said homeoprotein is a homeoprotein of a vertebrate.

The homeoprotein for use according to the invention is capable of protecting cells *in vivo* from DNA damage, in particular DNA strand breaks, in particular DNA double strand breaks (DSBs), formation and chromatin remodeling, in particular chromatin relaxation, especially those that are induced by stress factors or ageing. Stress factors refer in particular to oxidative or ecototoxic stress.

The effect of the homeoprotein toward DNA damage is not restricted to neurons or a particular type of neuron (e.g. mesencephalic DA neurons or RGCs) and can thus apply to other type of cells, such as for example motoneurons, fibroblasts, epithelial cells.

The following examples are not according to the invention and are present for illustration purposes only.

As used herein, the term "DNA-damage-related disorders" includes neurodegenerative disorders and diseases, such as cancer, aging and other disorders caused by damage to DNA due to exposure to oxidative stress, carcinogens, toxins, free radicals such as oxygen radicals, or DNA damaging radiation such as ionizing radiation and UV radiation. Although an understanding of mechanism is not required for practice of the invention, it is believed that administration of homeoprotein according to the present Invention prevents DNA damage, inhibits the effects of DNA damage, reduces the number of DNA strand breaks and/or stimulates a cellular repair response to DNA damage.

DNA damage may take the form of chromosomal dysfunction such as DNA strand breaks, or chemical modification of DNA (e.g. alkylation). According to preferred embodiment, DNA damage means DNA strand breaks, including single and double-strand breaks, preferably double-strand breaks.

According to the claimed invention, the term "DNA-damage-related disorders" relates to Parkinson's diseases. However, the important pathological feature is DNA damage. Thus, suppressing or limiting DNA damage and/or decreasing the amount of DNA strand breaks provides a method for decreasing neuronal cell death and/or treating /preventing neurological disorders associated to DNA damages.

The following aspect is not according to the invention and is present for illustration purposes only.

In another aspect, the description illustrates a method for treating DNA-damage-related disorder in a subject, the method comprising administering to a subject in need a composition comprising at least one homeoprotein, or peptide derivatives thereof, or a recombinant vector encoding said protein, and pharmaceutically acceptable vehicle.

As used herein, a "pharmaceutically acceptable vehicle" is intended to include any and all carriers, solvents, diluents, excipients, adjuvants, dispersion media, coatings, antibacterial and antifungal agents, and absorption delaying agents, and similar, compatible with pharmaceutical administration.

The composition of the invention is suitably buffered in order to be appropriate for human use at a physiological or slightly basic pH (e.g. from approximately pH 7 to approximately pH 9). Suitable buffers include without limitation phosphate buffer (e.g. PBS), bicarbonate buffer, HEPES and PIPES buffers and/or Tris buffer. The composition of the invention can further comprise a diluent appropriate for human or animal use. It is preferably isotonic, hypotonic or weakly hypertonic and has a relatively low ionic strength. Representative examples include sterile water, physiological saline (e.g. sodium chloride), Ringer's solution, glucose, trehalose or saccharose solutions, Hank's solution, and other aqueous physiologically balanced salt solutions (see for example the most current edition of Remington: The Science and Practice of Pharmacy, A. Gennaro, Lippincott, Williams & Wilkins). Pharmaceutically acceptable vehicles included in the composition of the invention must also allow the preservation of its stability under the conditions of manufacture and long-term storage (i.e. at least one month with a preference for at least one year) at freezing (e.g. -70 deg. C., -20 deg. C.), refrigerated (e.g. 4 deg. C.) or ambient temperatures. Additional pharmaceutically acceptable excipients may be used for providing desirable properties, including for example modifying or maintaining the pH, osmolarity, viscosity, clarity, colour, sterility, stability, rate of dissolution of the formulation, modifying or maintaining release or absorption into an the human or animal organism, promoting transport across the blood barrier or penetration in a particular organ (e.g. brain).

As understood by a person skilled in the medical art, the terms, "treat" and "treatment," refer to medical management of a disease, disorder, or condition of a subject (i.e., patient). In general, an appropriate dose and treatment regimen provide the homeoprotein in an amount sufficient to provide therapeutic and/or prophylactic benefit. Therapeutic benefit for subjects to whom the homeoprotein described herein are administered, includes, for example, an improved clinical outcome, wherein the object is to prevent or slow or retard an undesired physiological change associated with the disease, or to prevent or slow or retard the expansion or severity of such disease. As discussed herein, effectiveness of the one or more homeoprotein(s) may include beneficial or desired clinical results that comprise, but are not limited to, abatement, lessening, or alleviation of symptoms that result from or are associated with the disease to be treated; decreased occurrence of symptoms; improved quality of life; longer disease-free status (i.e., decreasing the likelihood or the propensity that a subject will present symptoms on the basis of which a diagnosis of a disease is made); reduction of disease extent; stabilized (i.e., not worsening) state of disease; delay or slowing of disease progression; amelioration or palliation of the disease state; and remission (whether partial or total), whether detectable or undetectable; and/or overall survival. The effectiveness of the homeoprotein described herein may also mean prolonging cell survival, more particularly of neurons, when compared to expected cell survival if a subject were not receiving the homeoprotein according to the Invention.

Subjects in need of treatment include those who already have the disease or disorder as well as subjects prone to have or be at risk of developing the disease or disorder, and those in which the disease, condition, or disorder is to be treated prophylactically. A subject may have predisposition for developing a disease or disorder that would benefit from the method of the Invention or may be of a certain age wherein receiving the method of the Invention would provide clinical benefit delaying development or reducing severity of a disease, including DNA damage-related disease or disorder and/or an age-related disease or disorder.

The dose of homeoprotein according to the Invention for treating DNA damage-related disease or disorder and/or an age-related disease or disorder may depend upon the subject's condition, that is, stage of the disease, severity of symptoms caused by the disease, general health status, as well as age, gender, and weight, and other factors apparent to a person skilled in the medical art. Pharmaceutical compositions may be administered in a manner appropriate to the disease to be treated as determined by persons skilled in the medical arts. In addition to the factors described herein and above related to use of homeoprotein for treating DNA damage-related disease or disorder and/or an age-related disease or disorder, suitable duration and frequency of administration of the homeoprotein may also be determined or adjusted by such factors as the condition of the patient, the type and severity of the patient's disease, the particular form of the active ingredient, and the method of administration. Optimal doses of the homeoprotein may generally be determined using experimental models and/or clinical trials. The optimal dose may depend upon the body mass, weight, or blood volume of the subject. The use of the minimum dose that is sufficient to provide effective therapy is usually preferred. Design and execution of pre-clinical and clinical studies for a homeoprotein (including time of administration for prophylactic benefit) described herein are well within the skill of a person skilled in the relevant art. When two or more homeoproteins are administered, the optimal dose of each homeoprotein may be different, such as less, than when either agent is administered alone as a single agent therapy. Typically, said homeoprotein may be used at concentrations ranging from 0.001 to 100 µM, or more preferable from 0.005 to 100 µM, advantageously from 0.005 to 10 µM, and particularly advantageously from 0.001 to 0.10 µM. An amount of homeoprotein that may be administered per day may be, for example, between 0.01 mg/kg and 100 mg/kg (e.g., between 0.1 to 1 mg/kg, between 1 to 10 mg/kg, between 10-50 mg/kg, between 50-100 mg/kg body weight). In other embodiments, the amount of an homeoprotein that may be administered per day is between 0.01 mg/kg and 1000 mg/kg, between 100- 500 mg/kg, or between 500-1000 mg/kg body weight. For intracerebral injection, the homeoprotein can be administered at at a dose comprised between 10 pmol and 100 pmol, for example at 25 pmol especially for Engrailed, as described in Rekaik et al., (2015, Cell Reports). In another embodiment, the homeoprotein can be administered by intraocular injection, especially for Otx2.

The optimal dose (per day or per course of treatment) may be different for the DNA damage-related disease or disorder and/or an age-related disease or disorder to be treated and may also vary with the route of administration and/or therapeutic regimen.

The pharmaceutically acceptable carriers are those conventionally used.

Thus, the invention concerns the Engrailed protein for use in the treatment or prevention of DNA-damage in a patient suffering Parkinson disease wherein Engrailed is administered once in the SNpc region of a subject in need thereof.

In addition, the administration of the homeoprotein may be combined with a non-pharmacological therapy. For example, it can be combined with subthalamic nucleus stimulation, for treating Parkinson Disease.

In addition to the above arrangements, the description also illustrates other arrangements, which will emerge from the description, which refers to exemplary embodiments, with reference to the Figures in which:

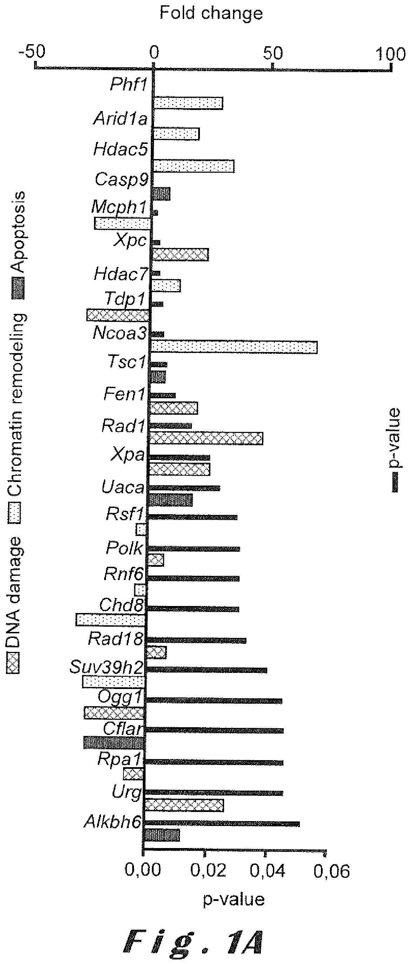

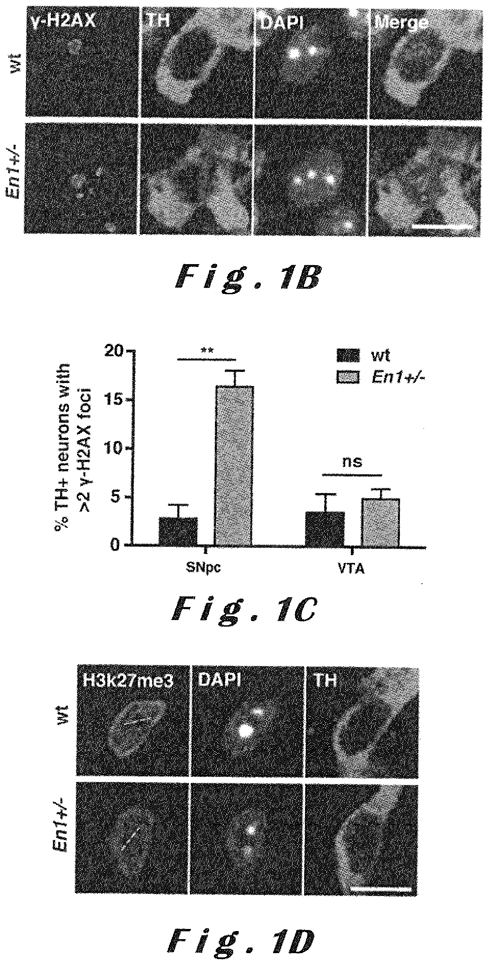

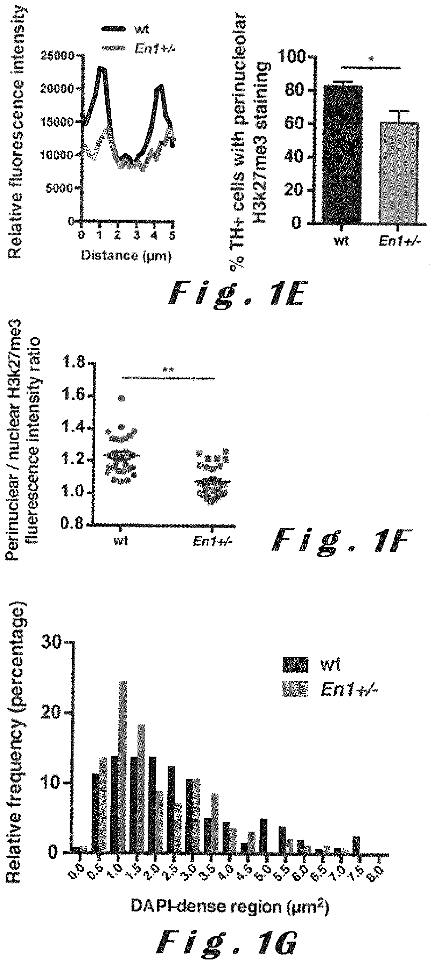

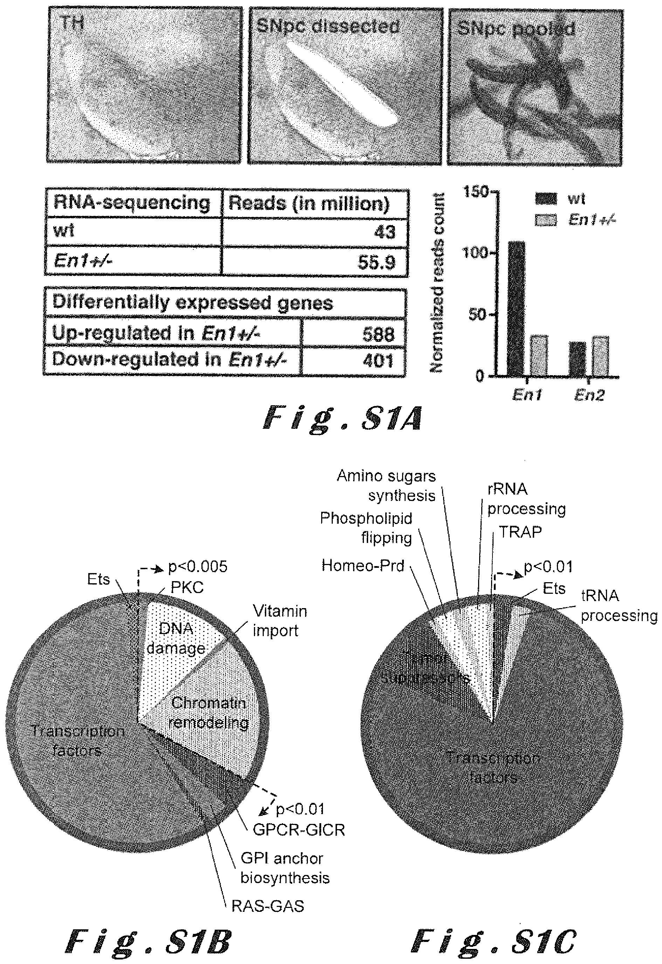

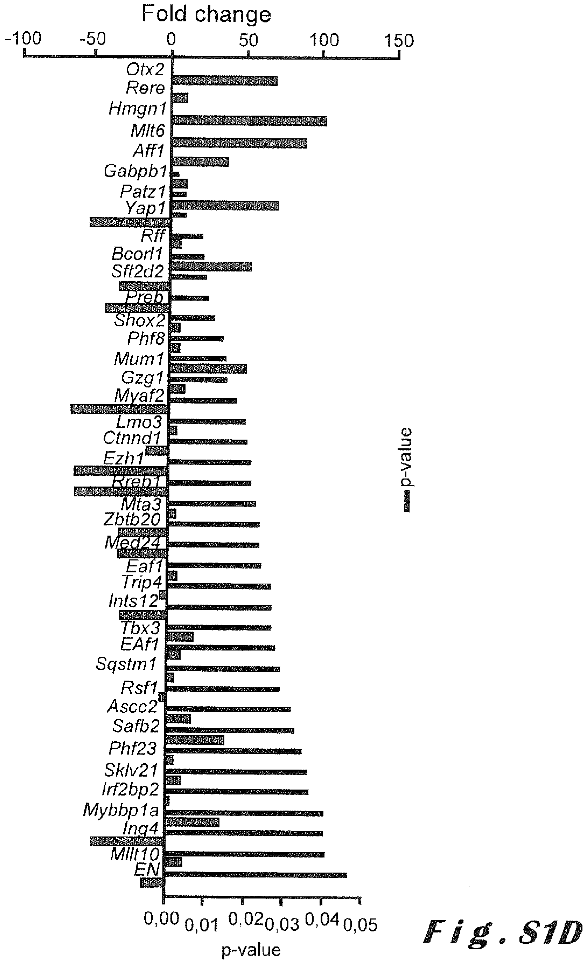

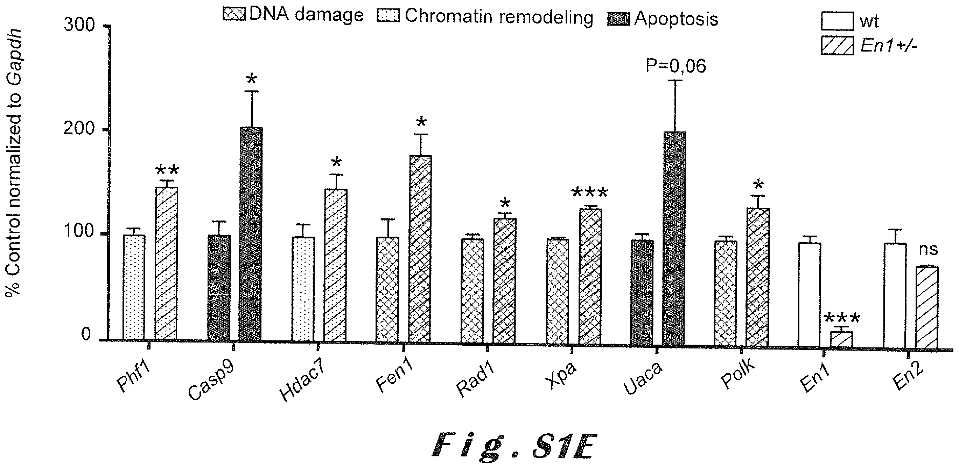

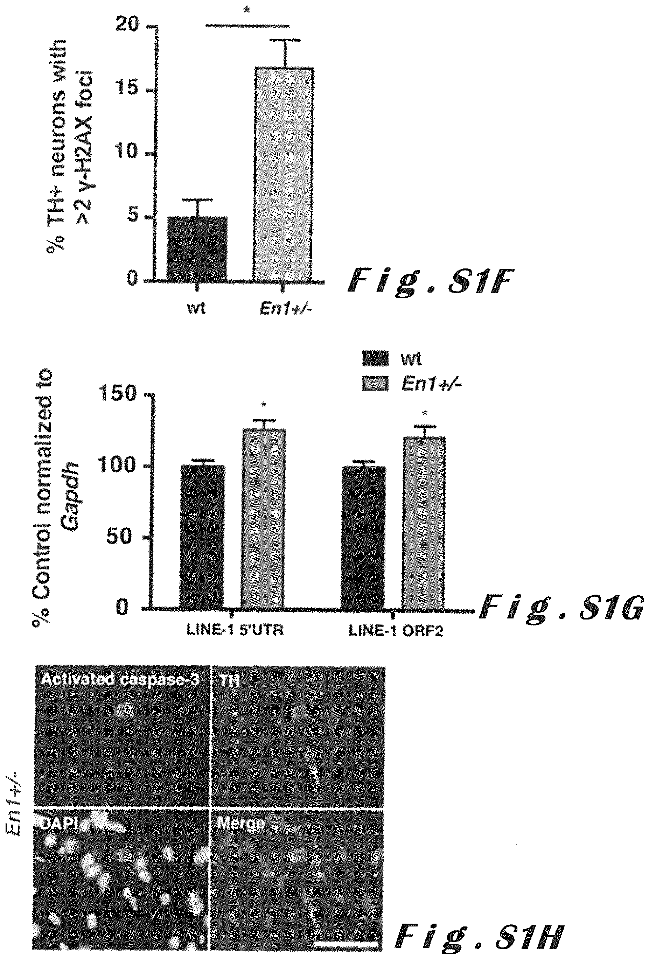

**Figure 1****.** Altered expression of DNA damage and heterochromatin marks in *En1+*/*-* mice
   (A) The differentially expressed genes in *En1+*/*-* SNpc TH+ neurons belonging to DNA damage, chromatin remodeling and apoptosis groups are ranked by p-values.
   (B) TH+ neurons in the SNpc of wt mice display a single ring of γ-H2AX staining ; *En1+*/*-* TH+ neurons show additional γ-H2AX foci scattered in the nucleus. Scale bar, 10 µm.
   (C) The percentage of TH+ neurons with >2 γ-H2AX foci in the SNpc increases from 2% in wt to 16% in 8 week-old *En1+*/*-* mice (n = 3; Student's t test). γ-H2AX staining in TH+ neurons in the VTA of *En1+*/*-* and wt mice is similar. Between 110 and 162 neurons counted in each condition.
   (D) Perinucleolar and perinuclear H3K27me3 staining is decreased in the *En1+*/*-* SNpc mDA neurons. Scale bar, 10 µm.
   (E) H3K27me3 perinucleolar staining is quantified by measuring fluorescence intensity (left) along the dotted lines. The percentage of cells with dense staining drops (right) in *En1+*/*-* mice (n = 3; Student's t test; 129 and 159 neurons counted in wt and *En1+*/*-* mice, respectively).
   (F) Perinuclear H3K27me3 staining in *En1+*/*-* TH+ neurons is reduced as shown by the decreased nuclear lamina/nucleoplasm fluorescence intensities (n = 3; Student's t test; 30 and 30 neurons counted in sham and 6-OHDA conditions).
   (G) Surface quantification of DAPI-dense regions. Frequency distribution indicates a shift towards smaller DAPI-dense areas in *En1+*/*-* mice (n=162-211; Kolmogorov-Smirnov test; 3 wt and *En1+*/*-* mice analyzed). See also **Figure S1** and **Table S1.**

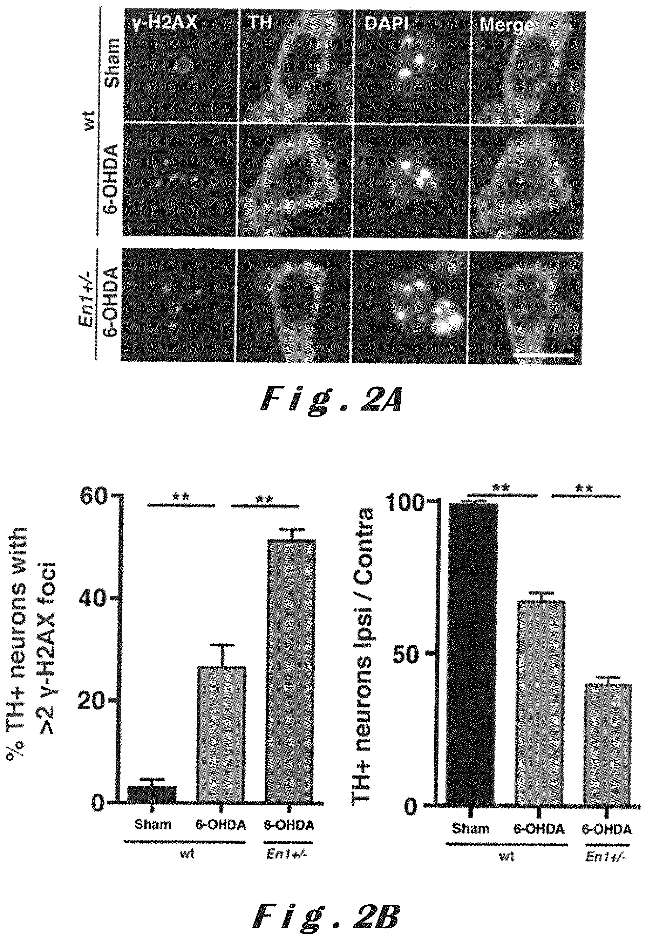

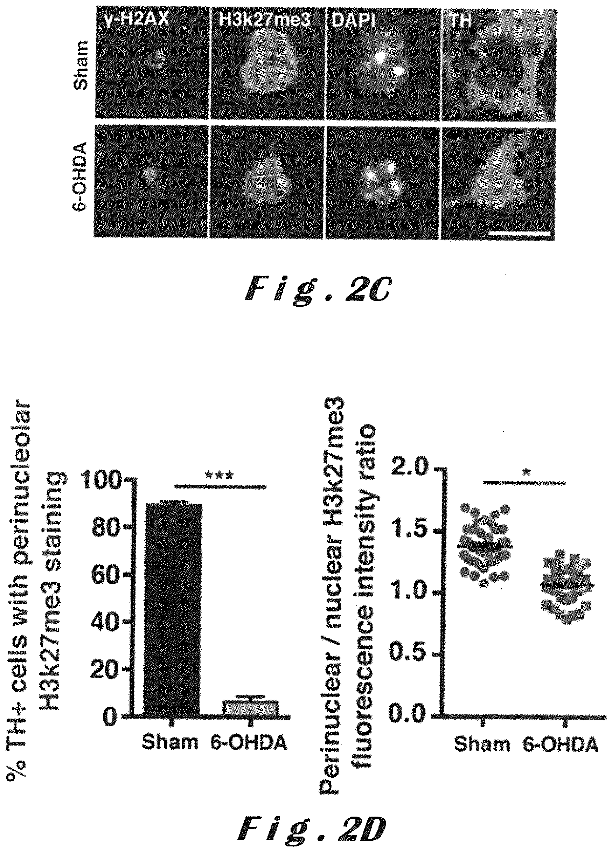

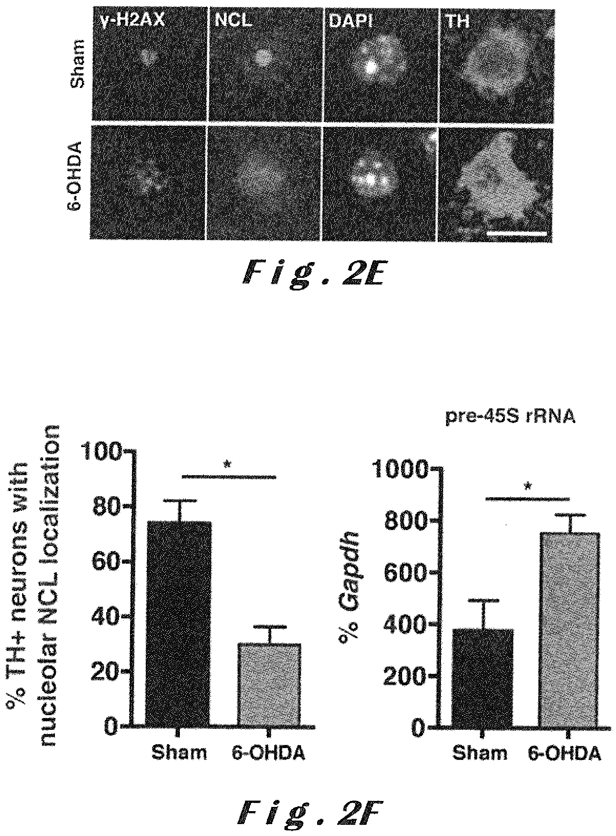

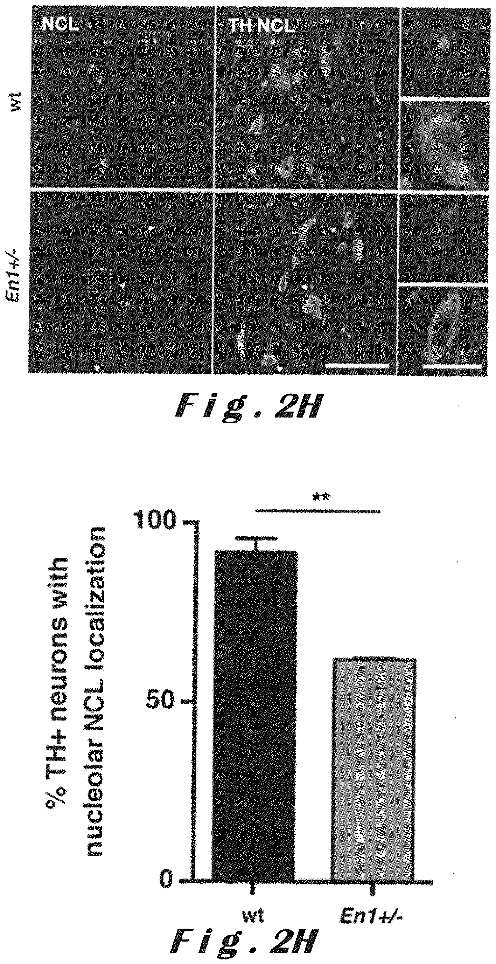

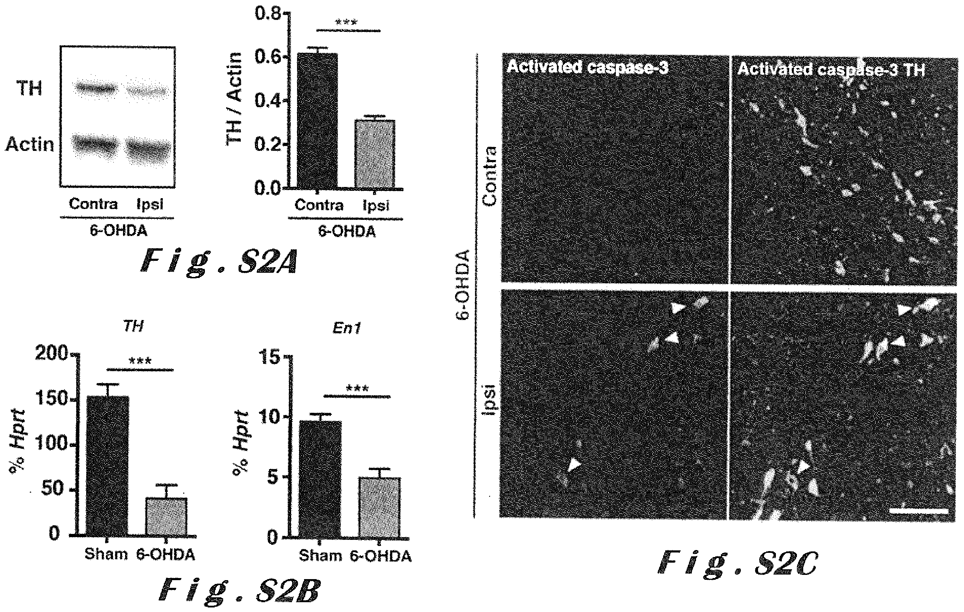

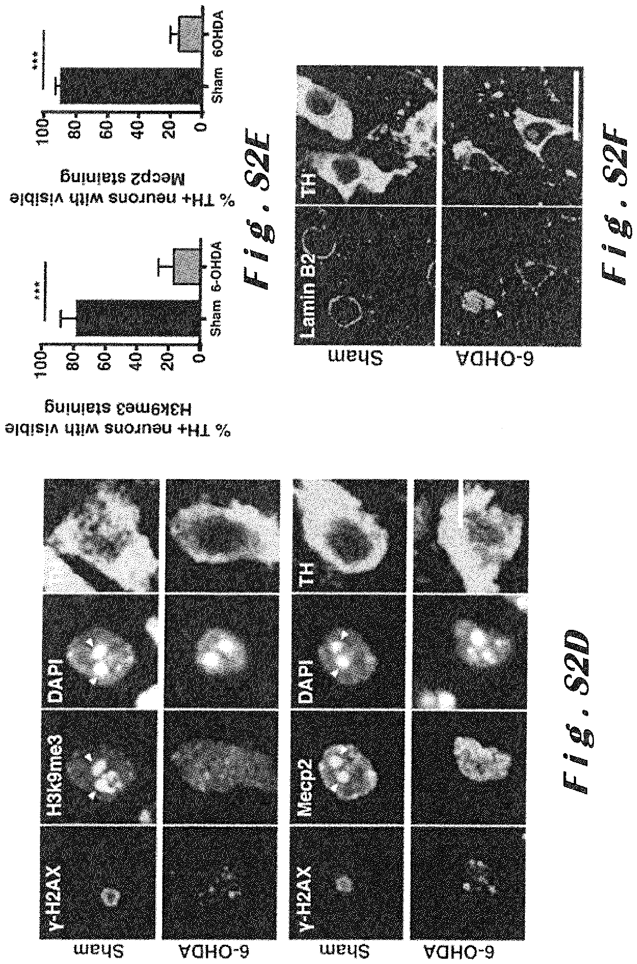

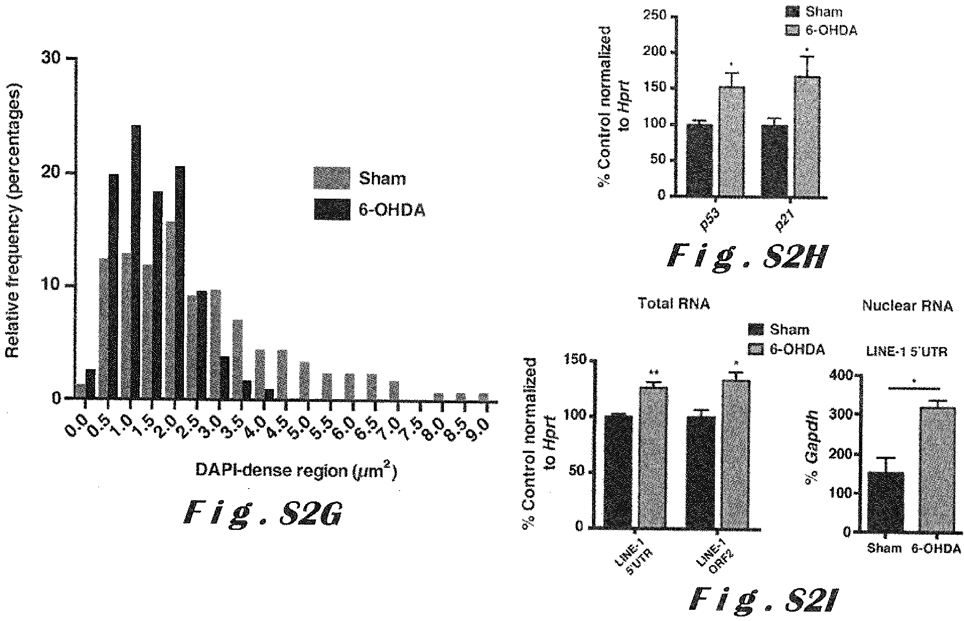

**Figure 2****.** DNA damage and chromatin alteration in SNpc TH+ neurons upon 6-OHDA injection
   (A,B) 6-OHDA injected in the SNpc of wt mice leads 6h later to the appearance of γ-H2AX foci in about 25% of TH+ neurons (n = 3-6; Student's t test). *En1+*/*-* mice are more sensitive with 50% of neurons showing multiple γ-H2AX foci (n = 3-6; Student's t test; 130, 210 and 146 neurons counted for each condition, respectively). Scale bar, 10 µm.6-OHDA injection provokes the loss of about 30% and 60% of TH+ neurons in wt and *En1+*/*-* mice, respectively (n = 3; Student's t test). The contralateral non-injected side is taken as a reference. In each condition between 1534 and 2034 neurons were counted.
   (C) Midbrain sections stained for γ-H2AX, H3k27me3 and TH and analyzed by confocal microscopy. Perinucleolar and perinuclear H3K27me3 staining is decreased upon 6-OHDA injection. Mice were analyzed 6h post injection. Scale bar, 10 µm.
   (D) The percentage of TH+ neurons displaying dense H3K27me3 perinucleolar and perinuclear staining quantified as in **Figures 1E****,F** drops dramatically (left) in 6-OHDA-injected mice (n = 3; Student's t test; 148 and 91 neurons counted in sham and 6-OHDA conditions, respectively). Perinuclear H3K27me3 staining in TH+ neurons also decreases upon 6-OHDA injection (right) (n = 3; Student's t test; 40 and 47 neurons counted in sham and 6-OHDA conditions, respectively).
   (E) Midbrain sections stained for γ-H2AX, nucleolin and TH and analyzed by confocal microscopy. Nucleolin nucleolar localization in sham-injected mice is lost 6 h after 6-OHDA-injection. NCL, nucleolin. Scale bar, 10 µm.
   (F) The percentage of TH+ neurons with nucleolar nucleolin is significantly decreased upon 6-OHDA injection (n = 3; Student's t test; 161 and 97 neurons were counted in sham and 6-OHDA conditions, respectively). NCL, nucleolin.
   (G) pre-45S rRNA analyzed by qRT-PCR is up-regulated following 6-OHDA injection in SNpc purified nuclei (n = 3; Student's t test).
   (H) Midbrain sections from (1 year-old animals) stained for nucleolin and TH were analyzed by confocal microscopy. Nucleolin presents a nucleolar localization in wt TH+ neurons whereas 40% of TH+ neurons in *En1+*/*-* mice present a diffuse staining pattern (arrows). Scale bar, 50 µm. Higher magnification images of dotted square areas are shown in the right most panels. Scale bar, 10 µm.The percentage of TH+ neurons with nucleolar nucleolin is significantly decreased in *En1+*/*-* mice (n = 3; Student's t test; 99 and 87 neurons counted in wt and *En1+*/*-* mice, respectively). NCL, nucleolin. See also **Figure S2.**

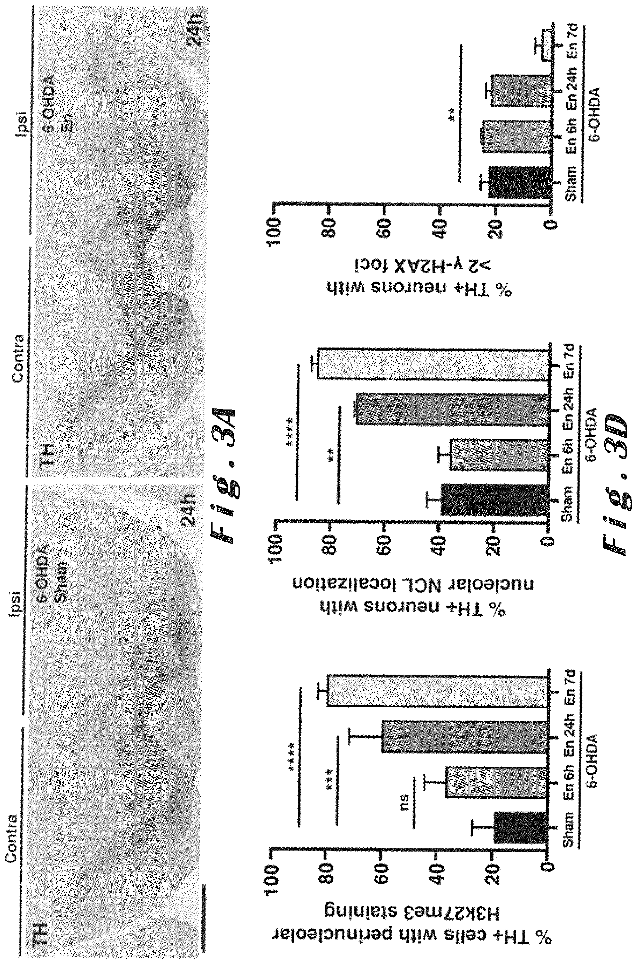

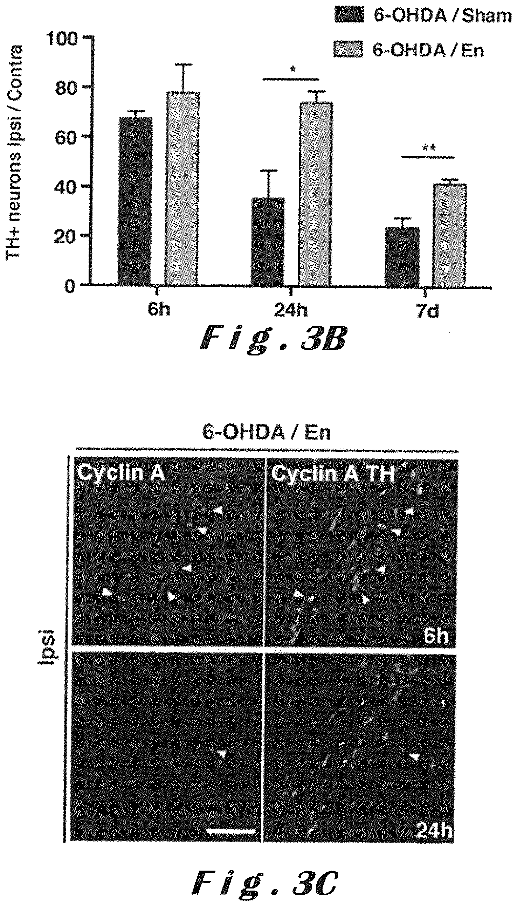

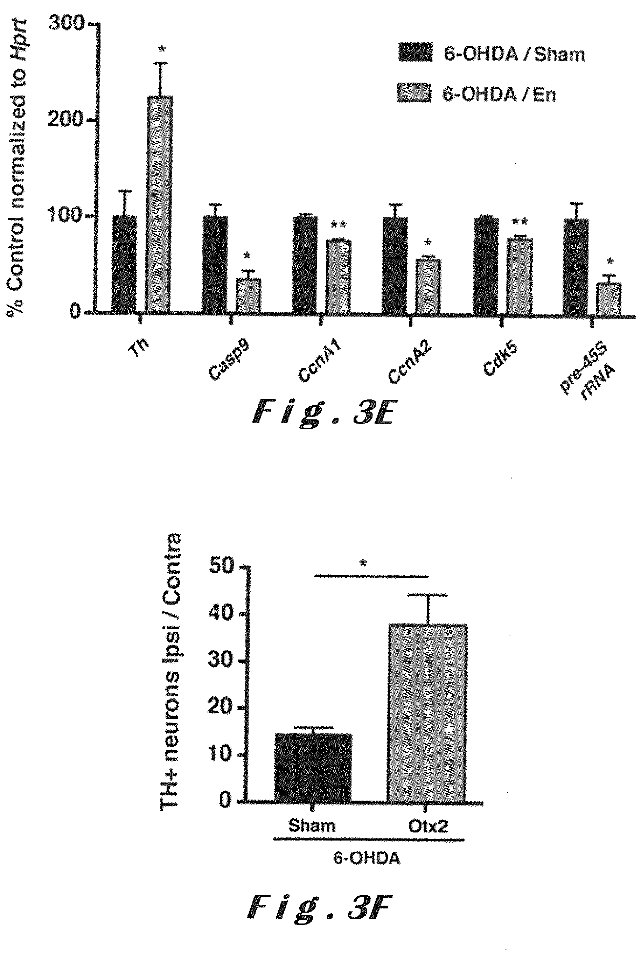

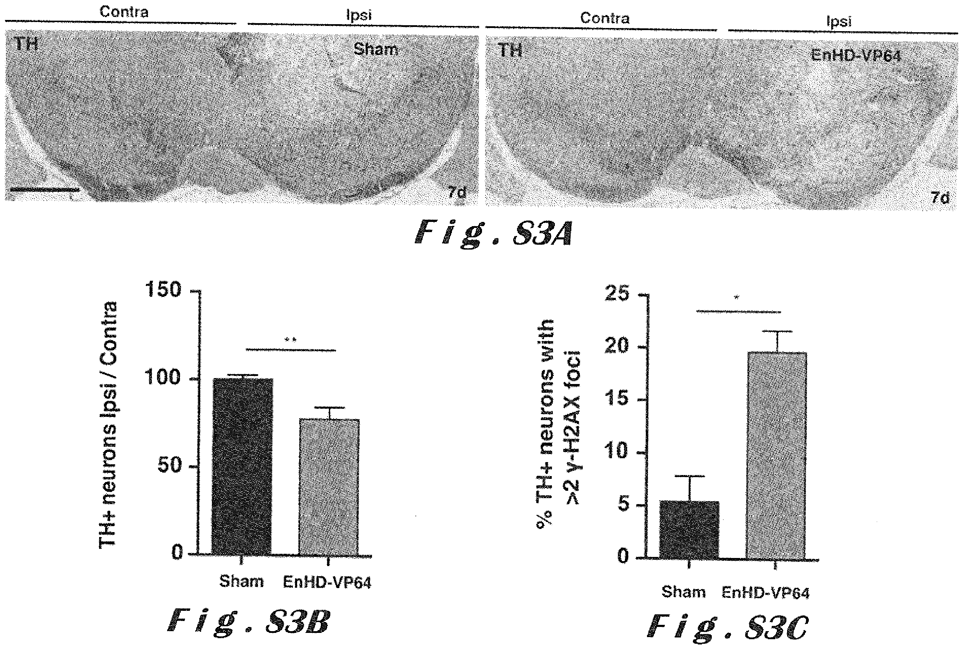

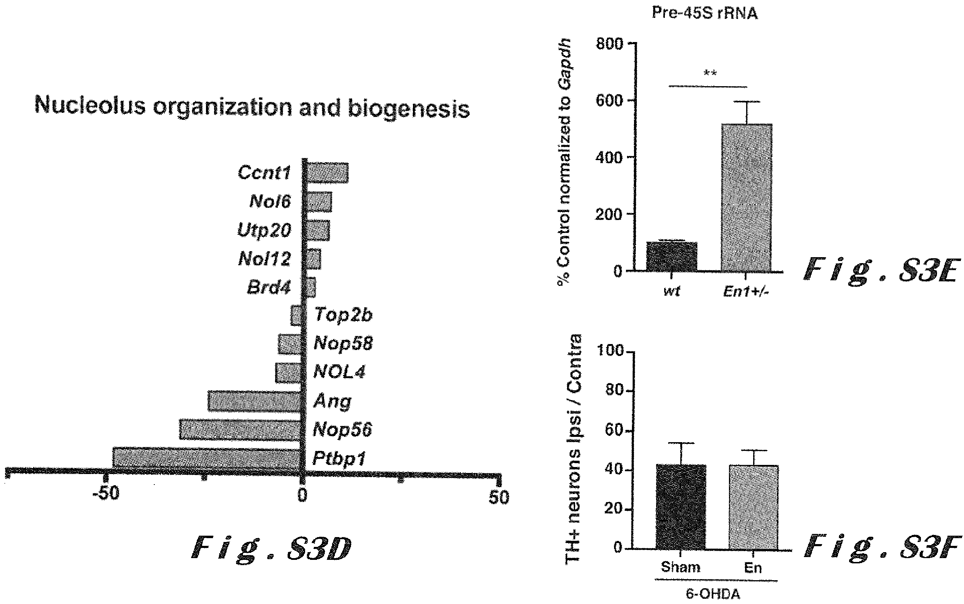

**Figure 3****.** Engrailed rescues TH+ cells from 6-OHDA-induced cell death
   (A) Mice injected with 6-OHDA in the SNpc were re-injected 30 min later with either sham or En2 and analyzed at 24h. Compared to sham, En2 injection prevents the 6-OHDA-induced TH cell loss in the ipsilateral SNpc. Scale bar, 500 µm.
   (B) Protective effect of En2 assessed by the ratio of TH+ cell counts in ipsilateral versus contralateral SNpc 6h, 24h and 7 days post-injection (n = 3-5; Student's t test). The number of neurons counted ranged between 1670 and 2275 per condition.
   (C) The rescue of TH+ neurons by En2 in 6-OHDA-injected mice is paralleled by a disappearance of cyclin A at 24h. Scale bar, 100 µm.
   (D) Immunostaining for H3K27me3 shows a progressive recovery following En2 injection. Recovery of nucleolar nucleolin is almost complete at 24h. The percentage of TH+ neurons with γ-H2AX foci returns to normal between 24h and 7 days after injection. For each analysis, n = 3. One way ANOVA followed by Dunetts's test (vs sham). The number of neurons counted ranged between 102 and 150 for each condition.
   (E) The expression of selected genes related to apoptosis and cell cycle in the SNpc of 6-OHDA/sham and 6-OHDA/En2 mice analyzed 6h post injections (n = 5; Student's t test).
   (F) Otx2 protects TH+ neurons against 6-OHDA-induced cell death. Otx2 was injected 30 min after 6-OHDA injection and mice analyzed 24h later. Protective effect was assessed as for En2 injections (n = 3; Student's t test). Number of neurons counted ranged between 1268 and 1495 for each condition. See also **Figure S3.**

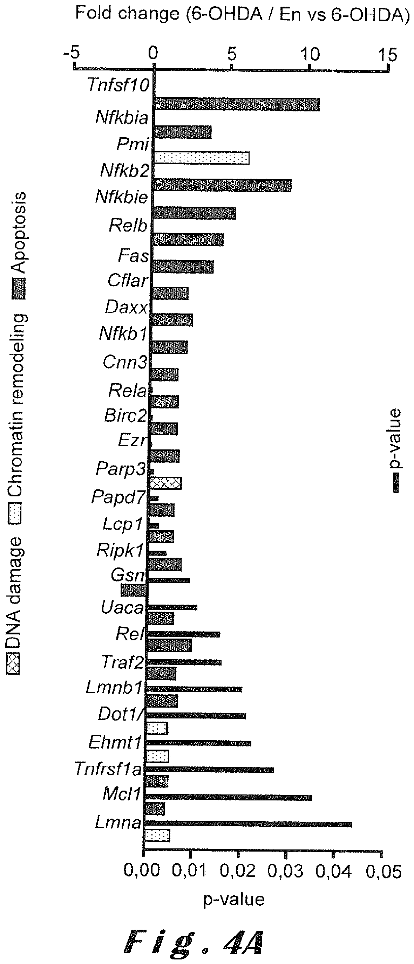

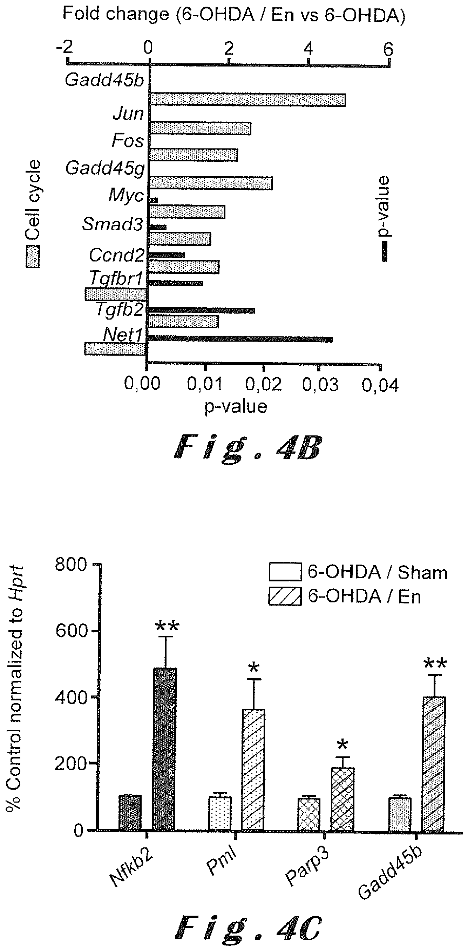

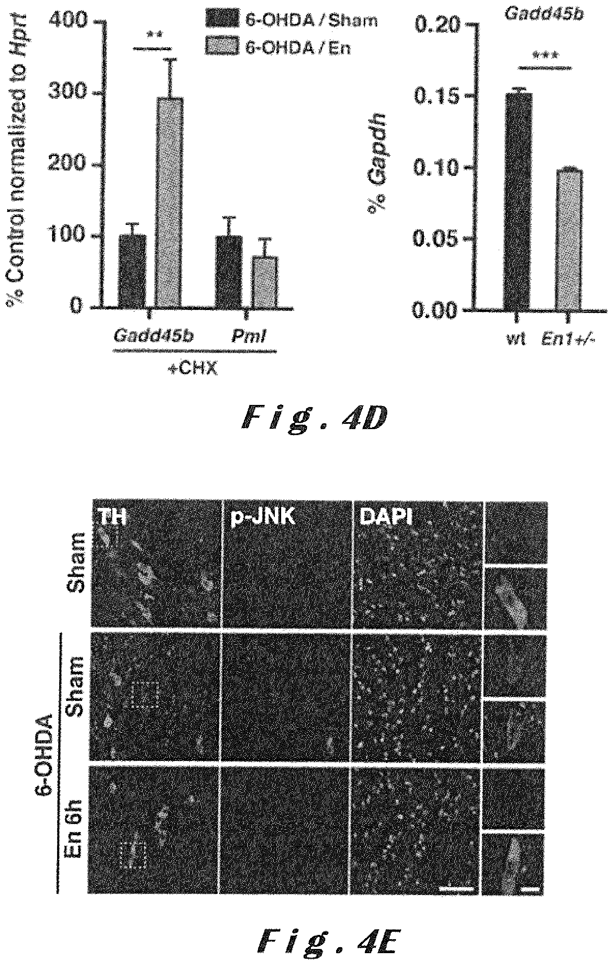

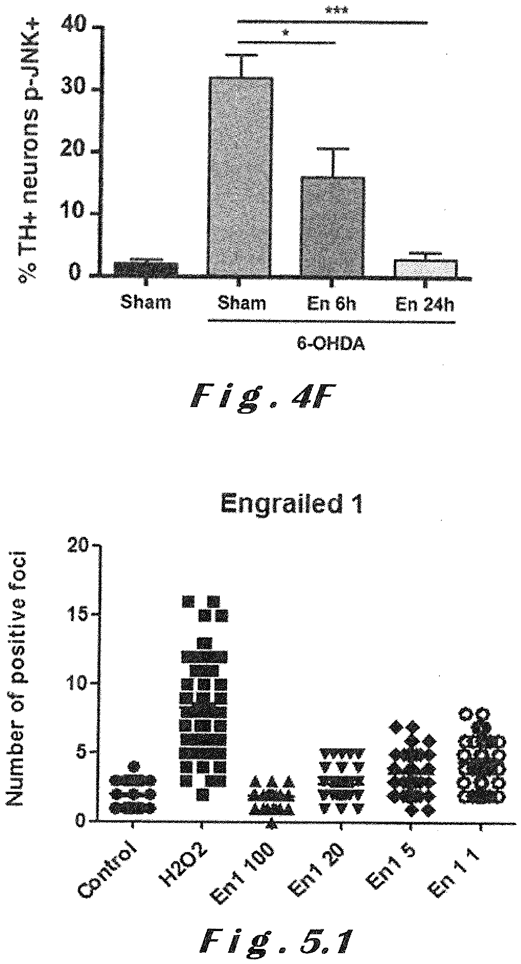

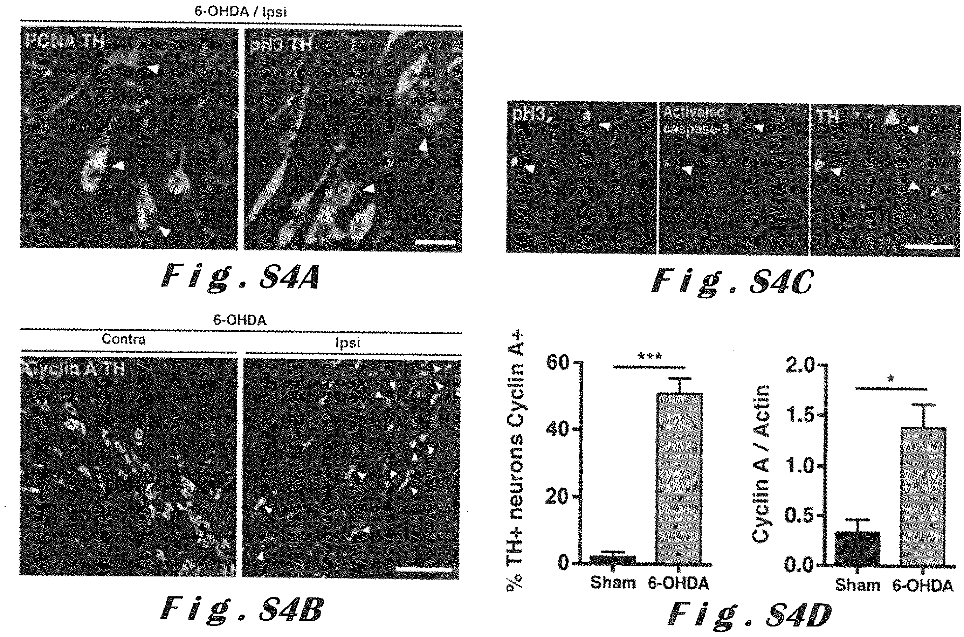

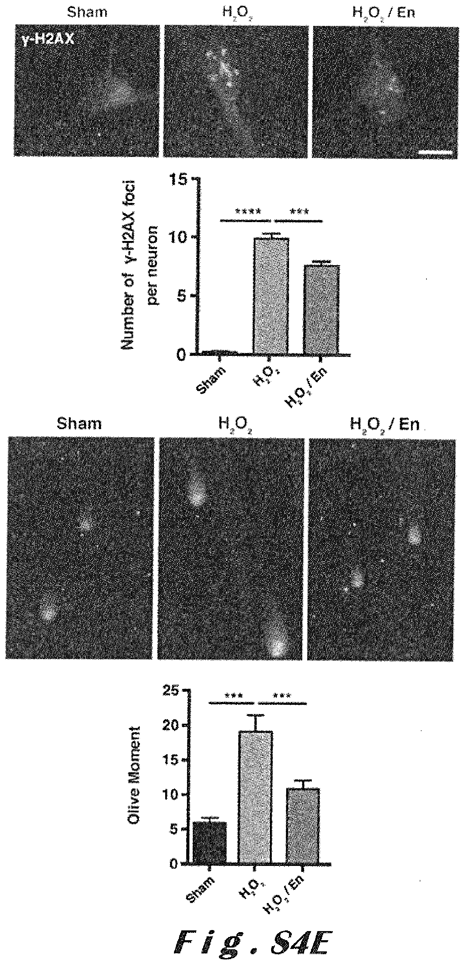

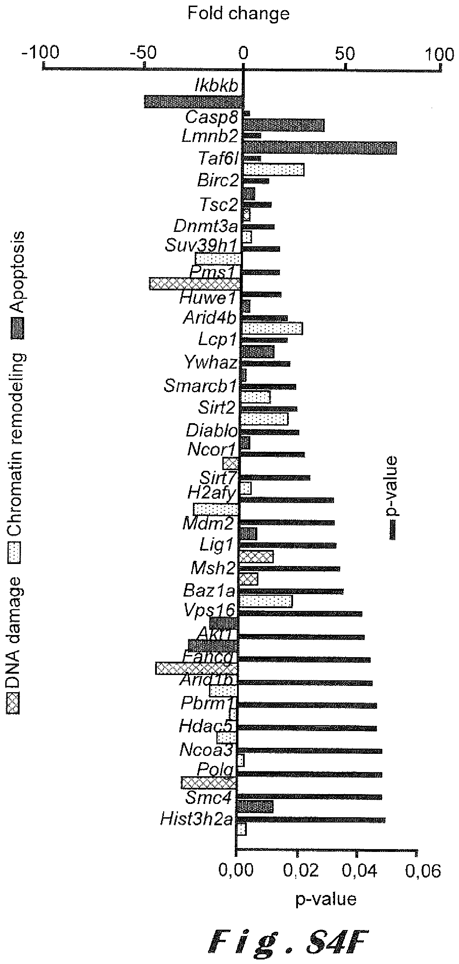

**Figure 4****.** RNA-seq analysis reveals Engrailed anti-apoptotic activity
   (A,B) Differentially expressed genes in the SNpc of 6-OHDA versus 6-OHDA/En2 related to DNA damage, chromatin remodeling, apoptosis and cell cycle are ranked by p-values.
   (C) The expression of selected genes in the SNpc of 6-OHDA and 6-OHDA/En2 is confirmed by qRT-PCR (n = 5; Students t test).
   (D) Left panel: *Gadd45b* and *Pml* transcripts were measured by qRT-PCR in the SNpc of 6-OHDA and 6-OHDA/En2 at 6h (injection with cycloheximide, CHX; n = 5; Student's t test). Right panel: *Gadd45b* transcripts in the SNpc of 8 week-old wt and *En1+*/*-* mice (n = 4; Student's t test).
   (E) Midbrain sections from sham, 6-OHDA/sham and 6-OHDA/En2 stained for p-JNK and TH and analyzed by confocal microscopy. Scale bar, 50 µm. Higher magnification images of dotted squares are shown in the right most panels. Scale bar, 10 µm.
   (F) En2 significantly decreases the percentage of SNpc TH+ neurons with p-JNK staining (n = 3; one way ANOVA followed by Tukey's multiple comparisons test; number of neurons analyzed ranged from 227 and 351 for each condition). See also **Figure S4.**
**Figure S1 (related to** **Figure 1****).** Altered genes expression in mDA neurons of *En1+*/*-* mice
   (A) SNpc from wt and *En1+*/*-* mice was laser microdissected for RNA-seq analysis. Total number of reads and differentially expressed genes (p < 0.05) are indicated. Decreased expression of *En1* in *En1+*/*-* SNpc was confirmed by RNA-seq.
   (B) RNA-seq data analysis (Pathway Studio) reveals a significant enrichment (p < 0.005) in Ontology gene subgroups involved in DNA damage and chromatin remodeling between wt and *En1+*/*-* littermates. Analysis using Cell Process Pathways also showed differential expression of apoptosis-related genes.
   (C) Analysis of RNA-seq data from Engrailed gain of function experiments by Pathway Studio. The gene subgroups enriched (p < 0.01) are indicated (n = 5).
   (D) Differentially expressed transcription factors in RNA-seq analysis by Pathway Studio of wt and *En1+*/*-* SNpc are ranked by p-value.
   (E) Altered expression of a selection of genes related to DNA damage, chromatin remodeling and apoptosis is confirmed by qRT-PCR using SNpc RNAs from 6 week-old wt and *En1+*/*-* mice (n = 3-6; Student's t test).
   (F) The percentage of TH+ neurons with >2 γ-H2AX foci in the SNpc increases from 5% in wt to 17% in 24 week-old *En1+*/*-* mice (n = 3; Student's t test; 107 and 94 neurons were counted in wt and *En1+*/*-* conditions, respectively).
   (G) LINE-1 transcripts in the SNpc of *En1+*/*-* mice analyzed by qRT-PCR increase, compared to wt (n = 5; Student's t test).
   (H) Activated caspase-3 positive TH+ neurons are detected in the SNpc of *En1+*/*-* mice (8 week-old). Scale bar, 50 µm.
**Figure S2 (related to** **Figure 2****).** TH cell death and chromatin changes in 6-OHDA injected mice
   (A) Western blot analysis shows a 50% decrease in TH protein levels at 6h in the 6-OHDA injected ipsilateral SNpc (n = 3).
   (B) The levels of mRNAs for TH and En1 are decreased (at 6h) by 70 and 50%, respectively in 6-OHDA injected SNpc (n = 6; Student's t test).
   (C) Activated caspase-3 is detected at 6h in TH+ neurons in the 6-OHDA-injected side but not in the contralateral side. Scale bar, 100 µm.
   (D) Triple immunostaining of midbrain sections for γ-H2AX, H3K9me3 and TH shows that H3K9me3 colocalization with DAPI in sham condition is lost in 6-OHDA-injected mice. Similarly, MeCP2 colocalization with DAPI in TH+ neurons of sham-injected mice is lost in 6-OHDA-injected mice. Scale bar, 10 µm.
   (E) The percentage of TH+ neurons with dense H3K9me3 or MeCP2 marks is significantly decreased in 6-OHDA-injected mice (n = 3; Student's t test). The numbers of neurons counted were 161 (sham) and 97 (6-OHDA) for H3K9me3 and 150 (sham) and 128 (6-OHDA) for MecP2.
   (F) Loss of perinuclear lamin B2 staining in TH+ neurons in the SNpc of 6-OHDA-injected mice. Scale bar, 20 µm.
   (G) Surface quantification of DAPI-dense regions in TH+ neurons in sham or 6-OHDA-injected mice. Compared to sham-injected controls, the distribution of the relative frequencies indicates a shift towards smaller DAPI-dense areas in TH+ neurons of 6-OHDA-injected mice (n = 137-177; Kolmogorov-Smirnov test; 3 mice per condition).
   (H) *p53* and *p21* mRNAs were quantified by qRT-PCR using total RNA from the SNpc of 6-OHDA or sham injected mice (n = 6; Student's t test).
   (I) Higher expression of repeat elements in 6-OHDA injected SNpc. The levels of LINE-1 transcripts analyzed by qRT-PCR using total SNpc RNA increase in 6-OHDA-injected mice. The level of LINE-1 RNA was also analyzed using RNAs from SNpc purified nuclei (n = 3-5; Student's t test).
**Figure S3 (related to** **Figures 2-3****).** Engrailed transcription activity involved in TH neuron protection
   (A) Mice were unilaterally infused with sham or EnHD-VP64 in the SNpc and analyzed at 7 days. Immunostaining of midbrain sections for TH shows that EnHD-VP64 infusion (but not sham) provokes TH cell loss in the ipsilateral SNpc. Scale bar, 500 µm.
   (B) EnHD-VP64 induced TH cell loss was assessed by comparing the ratio of TH+ cell counts in ipsilateral versus contralateral SNpc at 7 days post infusion (n = 6; Student's t test). The number of neurons counted ranged between 1902 and 2118 per condition
   (C) Quantification of γ-H2AX staining shows that the percentage of TH+ neurons with >2 γ-H2AX foci in the SNpc increases upon EnHD-VP64 infusion (n = 3; Student's t test; 138 and 129 neurons were counted in sham and EnHD-VP64 conditions, respectively).
   (D) Analysis of RNA-seq data also reveals differentially expressed genes in Pathway Studio Ontology subgroups related to nucleolar organization and biogenesis in the SNpc of *En1+*/*-* mice (p < 0.1).
   (E) The level of pre-45S rRNA in the SNpc of wt and *En1+*/*-* mice (8 week-old animals) was analyzed by qRT-PCR (n = 4; Student's t test).
   (F) Engrailed promotes TH+ neurons survival in the 6-OHDA model. The survival effect of Engrailed was not observed if Engrailed was injected 24h after 6-OHDA injection (when the majority of TH+ neurons are lost) and recovery assessed 6h later (n = 3).
**Figure S4 (related to** **Figure 4****).** Detection of cell cycle markers in TH+ neurons upon acute oxidative stress *in vivo* and Engrailed-mediated protection against H₂O₂-induced DSBs *in vitro*
   (A) Detection of PCNA and pH3 cell cycle markers in TH+ neurons in the 6-OHDA-injected side. Scale bar, 20 µm.
   (B) Immunostaining of midbrain sections reveals TH+ neurons expressing cyclin A only in the ipsilateral side of 6-OHDA-injected mice. Mice were analyzed 6h post injection. Scale bar, 100 µm.
   (C) Detection of activated caspase-3 in TH+ neurons staining positive for pH3. Scale bar, 50 µm.
   (D) The percentage of TH+ neurons expressing cyclin A upon 6-OHDA injection was quantified (left, n = 3; Student's t test) and confirmed by Western blot analysis (right, n = 4; Student's t test).
   (E) Engrailed treatment protects midbrain neurons against H₂O₂-induced DSBs. Primary mesencephalic neuron cultures of E14.5 mouse embryos were treated with H₂O₂ at 5 µM after a 24h incubation with NaCl or En1 (15 nM). Cells were fixed 1h after H₂O₂ treatment, stained for γ-H2AX and the number of foci was quantified (n = 68-137; one way ANOVA followed by Tukey's multiple comparisons test). En2-mediated protection against H₂O₂-induced DSBs using midbrain neuron cultures was assessed by the comet assay performed according to Trevigen CometAssay kit instructions and analyzed with OpenComet plugin, scale bar, 100 µm. The number of neurons with a comet tail was quantified (n = 39-46; one way ANOVA followed by Tukey's multiple comparisons test).
   (F) Differentially expressed genes in the SNpc of En2 versus sham injected mice. Genes related to DNA damage, chromatin remodeling, apoptosis are ranked by p-values.

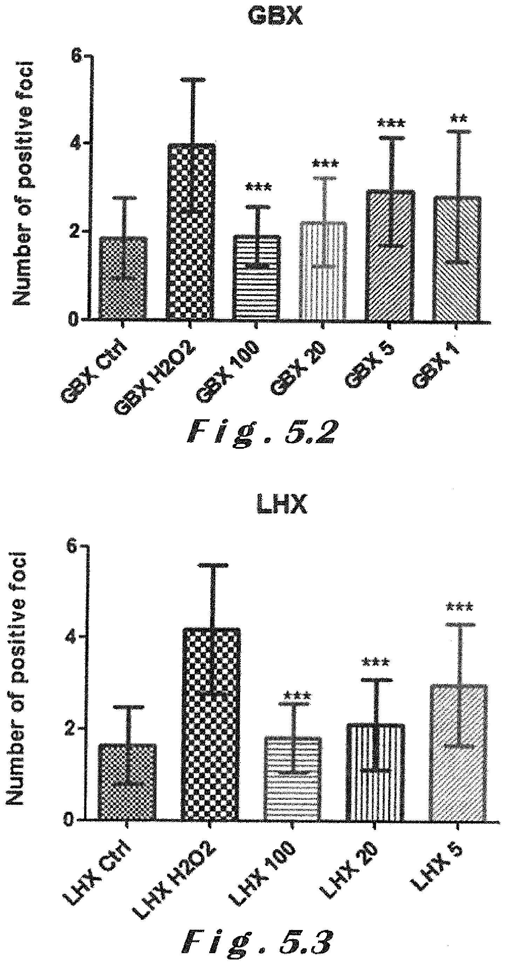

**Figure 5** **: En1/GBX2 /LHX9**
   5.1, Dose response for Engrailed ; 5.2, Dose response for Gbx2 ; 5.3, Dose response for Lhx9

### EXAMPLES

### EXPERIMENTAL PROCEDURES

### Animals

Mice were treated as per the guidelines for the care and use of laboratory animals (US National Institute of Health) and the European Directive 86/609 (EEC Council for Animal Protection in Experimental Research and Other Scientific Utilization). Swiss OF1 wt (Janvier) and *En1+*/*-* mice (Hanks et al., 1995 Science 269, 679-682) were maintained in conventional animal facility. Experimental groups consisted of 6 to 9 week-old mice.

### In vivo treatments

For 6-OHDA injections, mice were placed in a stereotaxic instrument and a burr hole drilled into the skull 3.3 mm caudal and 1 mm lateral to the bregma. The needle was lowered 4 mm from the surface of the skull and 6-OHDA (2 µl; 0.8 µg/µl Sigma) or sham (NaCl 0.9%) injections were performed over 4 min. This procedure targets the homeoprotein to the SNpc tissue. For Engrailed rescue experiments, a solution (2 µl) of bacterial recombinant En2 (300 ng; 4 µM) and colominic acid (3 µg) (Sonnier et al., 2007 J Neurosci 27,1063-1071) or vehicle (NaCl 0.9%) was injected 30 min after 6-OHDA injection using the same coordinates. When indicated, cycloheximide (0.1 µg/µl, sigma) was added. For Otx2 protein injection, a 2 µl solution containing 300 ng of the protein was used. Mice were killed at indicated times for analysis. SNpc tissues for qRT-PCR and Western blot analysis were obtained by performing 1 mm punches from 2 mm thick frozen coronal slices.

For EnHD-VP64, mice were infused for 7 days with an osmotic mini pump (Alzet 1002, Charles River Laboratories) connected to a 4 mm-long cannula placed at the same stereotaxic coordinates as above. The pump was filled with 100 µl containing En-VP64 (400 nM, 0.9% NaCl or the equivalent volume of an empty-plasmid-containing bacterial extract) and colominic acid (1.5 µg/µl).

### Image quantification

Images were analyzed with ImageJ. For immunofluorescence, all quantifications were performed using 60X magnification and 0.7 µm-thick successive focal planes. For H3K27me3 nucleolar pattern analysis, a graph of the intensities of pixels along a line positioned through the nucleolus was created. Perinuclear/nuclear ratio of H3K27me3 fluorescence intensity was determined by measuring pixel density at the periphery of the nucleus and in the nucleoplasm. DAPI-dense regions in sham and 6-OHDA injected mice were quantified by measuring individual DAPI-surface areas in each TH+ cell and plotting them as relative frequency distribution histograms.

### Statistical analysis

Statistical significance was determined using appropriate tests as indicated. Data are expressed as means ± SEM. *p < 0.5, **p < 0.01, ***p < 0.001, ****p < 0.0001 in all experiments.

### RNA-seq analysis

mDA neurons in the SNpc of wt and *En1+*/*-* mice labeled using the quick TH-staining protocol (Chung et al., 2005, Hum Mol Genet 14, 1709-1725) were isolated by Laser Capture Microdissection (LMD7000, Leica). Samples from 4 animals per group were pooled and total RNA was extracted using the AllPrep DNA/RNA Micro Kit (Qiagen) followed by DNase I using the RNeasy MinElute Cleanup protocol for on-column DNAse I treatment. Construction of cDNA libraries (Ovation RNA-seq System V2) and Illumina RNA-seq were performed by the Ecole Normale Supérieure Genomic platform (Paris). p-values of differentially expressed genes between wt and *En1+*/*-* samples were computed using DESeq package (Anders et al., 2010, Genome Biol 11, R106).. Normalized read counts were injected into the Gene Set Enrichment Analysis algorithm (Pathway Studio, Elsevier) to test for statistical enrichment. Pathway Studio Ontology and Cell Process Pathways collections were selected as Gene Set Categories for the analysis. RNA-seq analysis was also performed using RNA extracted from Laser Capture microdissected mDA neurons after Engrailed infusion (Alvarez-Fischer et al., 2011, Nat Neurosci 14, 1260-1266) in the SNpc of wt mice and from SNpc tissue punches collected 6h after 6-OHDA/sham and 6-OHDA/En2 injection.

### qRT-PCR

Total RNA from SNpc tissues was extracted using the RNeasy Lipid Tissue kit (Qiagen) followed by DNase I (Thermo) digestion. RNA (200 ng) was transcribed using the QuantiTect Reverse Transcription kit (Qiagen). qRT-PCR was performed using SYBR-Green (Roche Applied Science) and values normalized to *Gapdh* and/or *Hprt.* Data were analyzed using the ddCt method. In some experiments, RNA was isolated specifically from SNpc nuclei (Subcellular Protein Fractionation Kit, Thermo).

### Supplementary Methods Table : List of primers used for qRT-PCR analysis

### (refers to Experimental Procedures)

| **Gene** | Forward primer | Reverse primer |
|---|---|---|
| ***Casp9*** | TGGCTCCTGGTACATCGAGA SEQ ID NO. 5 | AGCATTGGCAACCCTGAGAA SEQ ID NO.6 |
| ***CcnA1*** | GGGCCTGCTCAGGCTGT SEQ ID NO. 7 | GTTCTCTGTGGGGGATCCTG SEQ ID NO. 8 |
| ***CcnA2*** | CTCGCTGCATCAGGAAGACC SEQ ID NO. 9 | TAAGAGGAGCAACCCGTCG SEQ ID NO. 10 |
| ***Cdk5*** | GCCAGACTATAAGCCCTACCC SEQ ID NO. 11 | GCTGCACAGGGTTACACTTC SEQ ID NO. 12 |
| ***En1*** | CCGGTGGTCAAGACTGACTC SEQ ID NO. 13 | CTGGTGCGTGGACCAGAG SEQ ID NO. 14 |
| ***En2*** | CCTTCTTCAGGTCCCAGGTC SEQ ID NO. 15 | AACTCAGCCTTGAGCCTCTG SEQ ID NO. 16 |
| ***Fen1*** | ATTCCTCTTCGCCGCCATT SEQ ID NO. 17 | GAGGCATCGATGGCCACTTT SEQ ID NO. 18 |
| ***Gadd45b*** | TCTCTAGAGGAACGCTGAGACC SEQ ID NO. 19 | GTAGGGTAGCCTTTGAGGGATT SEQ ID NO. 20 |
| ***Hdac7*** | CCCACCTGTCAGACCCAAGT SEQ ID NO. 21 | AGTCATAGACCAGCCCTGTAGCA SEQ ID NO. 22 |
| **LINE-1 5'UTR** | CTGGGAACTGCCAAAGCAAC SEQ ID NO. 23 | CCTCCGTTTACCTTTCGCCA SEQ ID NO. 24 |
| **LINE-1 ORF2** | AATCGACAAATGGGACCTAATGA SEQ ID NO. 25 | GTAAAGATCCTTTCCCAATCTGTTG SEQ ID NO. 26 |
| ***Nfkb2*** | GGTGGAAGACAAGGAGGAAG SEQ ID NO. 27 | AGGAGGAGAAAAAGCCGAG SEQ ID NO. 28 |
| ***p21 (Cdkn1a)*** | GGCCCGGAACATCTCAGG SEQ ID NO. 29 | A AATCTGTCAGGCTGGTCTGC SEQ ID NO. 30 |
| ***p53*** | GGCGTAAACGCTTCGAGATG SEQ ID NO. 31 | CTTCAGGTAGCTGGAGTGAGC SEQ ID NO. 32 |
| ***Parp3*** | GACCCCAGCTTGAAGAGTCC SEQ ID NO. 33 | CTGCCCATCCAGTTCGAGTT SEQ ID NO. 34 |
| ***Phƒ1*** | CTATCCGGATGTTCGCCTCC SEQ ID NO. 35 | GAGGTGACCTATCTGGGGGT SEQ ID NO. 36 |
| ***Pml*** | ATAGCAGCAGTGAGTCCAGC SEQ ID NO. 37 | GCTGGCTAATTTTCTGGGTTTCA SEQ ID NO. 38 |
| ***Polk*** | CACAGCACTTGCAGGAAAGG SEQ ID NO. 39 | CCTGTCTGGGTGTGTCGATT SEQ ID NO. 40 |
| **pre-45S rRNA** | CGTGTAAGACATTCCTATCTCG SEQ ID NO. 41 | GCCCGCTGGCAGAACGAGAAG SEQ ID NO. 42 |
| ***Rad1*** | TACTGCTTAGTGGCCAGCCT SEQ ID NO. 43 | AGGCATTTGCTTGCACACAC SEQ ID NO. 44 |
| **Th** | GTACTGGACAGTCCTCACACCA SEQ ID NO. 45 | GGTGGTACCCTATGCATTTAGC SEQ ID NO. 46 |
| **Uaca** | AGAGGTCAAGAAGGGCAAGC SEQ ID NO. 47 | TGCCACTCAACGCTCTTTCT SEQ ID NO. 48 |
| ***Xpa*** | GCGTGGCCAGTGTAAAAGCA SEQ ID NO. 49 | CGCATTCTTCACAGATGGTGT SEQ ID NO. 50 |

### Immunostaining

Mice were perfused with 4% paraformaldehyde in PBS, brains were postfixed for 1h and cryoprotected in 15% sucrose. Tissues embedded in Tissue-Tek O.C.T. (Sakura Finetek) were frozen isopentane prior to storage at -80°C. Brains were cut at the level of the SNpc into 18 µm-thick sections. For immunofluorescence, sections were permeabilized in 1% Triton X-100 in PBS for 20 min and incubated at 100°C for 20 min in citrate buffer (10 mM citric acid, 0.05% Tween 20, pH 6.0). After blocking for 1h (10% normal goat serum, 0,05% % Triton X-100 in PBS), tissues were incubated overnight at 4°C with primary antibodies diluted in the blocking solution (mouse anti-γ-H2AX, 1:200, Millipore; chicken anti-TH, 1:500, Abcam; rabbit anti-activated-caspase3, 1:200, Abcam; rabbit anti-nucleolin, 1:200, Sigma; rabbit anti-fibrillarin, 1:200, Cell Signaling; rabbit anti-H3k27me3, 1:200, Millipore; rabbit anti-H3k9me3, 1:200, Abcam; a generous gift from Edith Heard; rabbit anti-Lamin B2, 1:100, Santa Cruz; rabbit anti-Mecp2, 1:300, Abcam; rabbit anti-PCNA, 1:200, Cell Signaling; rabbit anti-cyclin A, 1:200, Santa Cruz; mouse anti-pH3, 1:100, Cell Signaling; mouse anti-p-JNK, 1:200, Santa Cruz). Sections were incubated with appropriate secondary antibodies (488 anti-chicken, 647 anti-chicken, 488 anti-mouse, 546 anti-mouse and 546 anti-rabbit Alexa Fluor, Life Technologies) for 1h at room temperature. Labeled sections were imaged by confocal microscopy (SP5, Leica). For TH immunohistochemistry, sections were permeabilized in 1% Triton X-100 and incubated overnight at 4°C with 10% normal goat serum in PBS containing a rabbit polyclonal antibody against TH (1:1000; Pel-Freez Biologicals). Sections were treated with biotinylated secondary antibody and incubated with avidin-biotinylated horseradish peroxidase complex (ABC system, Vectastain). Peroxidase was revealed using DAB peroxidase (HRP) substrate kit (Vectastain) and imaged with a Nokon Eclipe i90 microscope.

### RESULTS

### Example 1 : mDA neuron gene expression in the SNpc of En1+/- mice

RNA-seq analysis on microdissected SNpc was performed in wt and *En1+*/*-* mice. Comparable reads were obtained in wt and *En1+*/*-* mice with 989 differentially expressed genes (p < 0.05) **(Figure S1A).** Analysis was performed on 6 week-old animals when all neurons are still present in the in *En1+*/*-* mice. Pathway Studio Ontology (Gene Set Enrichment Analysis, Pathway Studio software) indicates that the three most represented and significant groups are DNA repair (p = 0.002), chromatin remodeling (p = 0.004) and transcription factors (p = 0.007); Cell Process Pathways analysis also revealed differential apoptosis regulation genes (p = 0.01) **(Figure S1B).** Genes within these ontologies and pathways were ranked by increasing p-values; **Figure 1A** highlights those with significant differences in read numbers.

Transcription factors genes represent the most abundant group **(Figure S1B); Figure S1D** ranks by p-values those with modified expression in *En1+*/*-* mice. En2 infusion in the SNpc of wt mice also modifies transcription factor genes, but very less so DNA damage response and chromatin modifying ones **(Figure S1C)** that were thus further investigated in the context of this study. Quantitative RT-PCR **(Figure S1E)** confirmed that 6 week-old *En1+*/*-* mice display altered expression of several genes related to DNA damage, chromatin remodeling and apoptosis.

### Example 2 : DNA strand breaks and modified chromatin marks in En1+/- mDA neurons

mDA neurons in the SNpc of *En1+*/*-* mice (between 6-8 weeks of age) were examined for signs of DNA damage by following the DNA strand breaks (DSBs) marker γ-H2AX (Lobrich et al., 2010 Cell Cycle 9, 662-669). This revealed the presence of multiple γ-H2AX foci in about 16% of TH+ neurons in the SNpc **(****Figures 1B,C****).** Of note, DSBs do not necessary lead to rapid cell death as shown by the absence of significant death, in the *En1+*/*-* mutant, between 24 and 48 weeks even though 16% of the cells have multiple γ-H2AX foci at 24 weeks **(Figure S1F).** In wt mice, about 98% of mDA neurons display a single ring of γ-H2AX around the nucleolus **(****Figure 1B****)** also present in neurons throughout the brain. In line with the lesser sensitivity of VTA mDA neurons to the loss of one *En1* allele, γ-H2AX staining was similar in the VTA of wt and *En1+*/*-* mice **(****Figure 1C****).**

*En1+*/*-* mice also have signs of chromatin alteration. As shown in **Figure 1D-F****,** the pattern of perinucleolar and perinuclear H3K27me3 (K27 trimethylated histone H3) staining is changed in a significant fraction of *En1+*/*-* TH+ neurons. The size distribution of the DAPI-dense regions of heterochromatin (Guenatri et al., 2004 J Cell Biol 166, 493-505) also shows a reduction in the percentage of large spots in the *En1+*/*-* mouse **(****Figure 1G****).** Heterochromatin changes are often associated with changes in the expression of Long Interdispersed Nuclear Elements (LINEs) accordingly LINE expression is increased in the SNpc of *En1+*/*-* mutants **(Figure S1G).** The induction of apoptosis genes **(****Figure 1A****)** is confirmed by the TH/activated caspase-3 co-staining in the SNpc of *En1+*/*-* mice, never seen in wt littermates **(Figure S1H).**

### Example 3 : Enhanced sensitivity of En1+/- mDA neurons to acute oxidative stress

An acute oxidative stress was applied to mDA neurons by injecting 6-OHDA (a superoxide producing drug specifically captured by mDA neurons) directly into the SNpc. This induced, within 6h, the loss of 35% of the TH+ neurons and the formation of multiple abnormal γ-H2AX foci in about 26% of the remaining ones **(****Figure 2A,B****),** only in the ipsilateral SNpc. Neuronal loss was paralleled by a reduction in TH protein and mRNA (*En1* mRNA also) **(Figures S2A,B)** and the presence of numerous activated caspase-3-positive mDA neurons **(Figure S2C).** Injecting 6-OHDA in *En1+*/*-* mice led to a higher percentage of TH+ neurons with γ-H2AX foci in the 6-OHDA-injected side (51%) and to a parallel increase in the loss of TH+ neurons (60%) **(****Figure 2A, B****).** EnHD-VP64 infusion indeed leads to mDA cell death **(Figure S3A,B)** and to an increase in the number of γ-H2AX foci **(Figures S3C).**

### Example 4 : Oxidative stress induces changes in heterochromatin marks

Heterochromatin marks are also modified 6h after 6-OHDA injection in wt mice. The dense perinucleolar and perinuclear H3K27me3 staining in TH+ neurons of sham-injected mice is changed into diffuse nucleoplasmic staining in 6-OHDA-injected mice **(****Figure 2C****).** This change was quantified in the nucleolus by measuring perinucleolar fluorescence intensity along one diameter **(****Figure 2D****,** left). Similarly, the ratio of H3K27me3 fluorescence intensities between the peripheral nuclear lamina and the nuclear stroma dropped from 1.4 to 1.0 **(****Figure 2D****,** right). Acute 6-OHDA also disrupted H3K9me3 (K9 trimethylated histone H3) and MeCP2 staining **(Figure S2D,E),** with the loss of neat lamin B2 staining **(Figure S2F)** and a change in the size distribution of DAPI-dense spots **(Figure S2G).** MeCP2 binds methylated CpGs and changes in its staining might reflect guanine oxidation.

Nucleolar stress, suggested by perinucleolar γ-H2AX loss upon 6-OHDA **(****Figure 2A****),** is verified by the drop from 70% (sham) to 30% (6-OHDA) of mDA cells with dense nucleolin staining **(****Figure 2E,F****).** This change was accompanied by a strong up-regulation of ribosomal pre-45S RNA **(****Figure 2G****)** signing nucleolar damage. In comparison, nucleolin staining, normal in *En1+*/*-* TH+ neurons at 6 weeks, shows signs of disruption at one year **(****Figure 2H****).** However, the expression of genes involved in nucleolus organization (RNA-seq) and qRT-PCR analysis of pre-45S rRNA suggest a change in nucleolar physiology in 6-8 week-old *En1+*/*-* mice **(Figure S3D,E).**

### Example 5 : Engrailed decreases the number of DSBs induced by H₂O₂

To verify if Engrailed is able to regulate DNA damage induced by oxidative stress, wt mice were unilaterally injected with 6-OHDA, and re-injected 30 min later with vehicle (sham) or En2. Analyses were done at 6h, 24h or 7 days post-injections. The dramatic loss at 24h of TH+ cells in the SNpc of 6-OHDA/sham injected is highly reduced in 6-OHDA/En2 injected mice **(****Figure 3A****).** Protection is still visible at 7 days with 40% and 20% of surviving neurons in En2-injected and sham mice, respectively **(****Figure 3B****).** En2 injection 24h (instead of 30 min) after 6-OHDA and analyzed 6h later showed no recovery, showing that TH staining corresponds to true survival and not to TH re-expression **(Figure S3F).**

6-OHDA-induced apoptosis is paralleled by the expression of cell cycle markers **(Figure S4A-D).** Accordingly, Cyclin A expression detected in En2-treated TH+ cells 6h post injections had almost disappeared at 24h **(****Figure 3C****).** Neuronal rescue by En2 is associated with the reappearance of normal staining patterns for H3K27me3, nucleolin and γ-H2AX **(****Figure 4D****).** The percentage of TH+ cells with "wild-type" perinucleolar H3K27me3 staining increased from 20% to 37% at 6h and reached 60% and 80% at 24h and 7 days, respectively. Wild-type nucleolin pattern took longer to reappear as recovery was observed only at 24h with little changes between 24h and one week. Finally the decrease in the number of γ-H2AX foci was slower with full recovery only at 7 days.

Rescue correlated with a significant increase in TH mRNA expression and a decrease in that of pre-45S rRNA already 6h after En2 injection **(****Figure 3E****).** The expression of selected genes related to cell cycle and apoptosis, up-regulated in the *En1+*/*-* mouse or upon 6-OHDA injection, was repressed in the SNpc of 6-OHDA/En2 mice **(****Figure 3E****).** Analyses were done at 6h when sham and En2-injected animals still have similar numbers of mDA neurons **(****Figure 3B****).**

E14.5 midbrain neurons expressing Engrailed but of which only 1-2% are dopaminergic were exposed to H₂O₂ with or without En2. **Figure S4E** illustrates that En2 decreases the number of DSBs induced by H₂O₂ in parallel with a decrease in the formation of comet tails that sign DNA damage.

### Example 6 : Engrailed activates short-and long-term survival pathways

Protection by En2 requires its injection before 24h **(Figure S3F).** To identify genes in this early survival pathway, SNpc RNA from 6-OHDA/sham or 6-OHDA/En2 mice (6h) was sequenced and genes in the chromatin remodeling, DNA damage, apoptosis and cell cycle pathways were analyzed according to Pathways Studio. **Figure 4A****,B** ranks by order of significance the genes with highest differences in read numbers and **Figure 4C** provides qRT-PCR confirmation of enhanced expression for top representative genes in the 4 analyzed pathways. Genes in the apoptosis pathway are more represented than in the *En1+*/*-* mouse **(****Figure 1****).** This is not due to En2 injection per se **(Figure S4F),** suggesting that the rapid up-regulation of anti-apoptotic pathways by En2 takes place specifically following the acute oxidative stress.

Paralleling *Gadd45b* decreased expression in 8 week-old *En1+*/*-* mice, the addition of cycloheximide in the rescue experiments demonstrates that *Gadd45b,* as opposed to *Pml* for control, does not require the translation of an intervening protein, and is thus a direct target of Engrailed, **(****Figure 4D****).** The high induction of *Gadd45b*/*g* and *NF-kB* suggested a role for c-Jun N-terminal kinase (JNK) signaling, a pathway implied in several neurodegenerative diseases, including PD (Coffey, 2014, Nat Rev Neurosci 15, 285-299). **Figure 4E****,F** confirms that the strong increase in p-JNK staining 6h after 6-OHDA is antagonized by En2 **(****Figure 4F****).**

### Example 7 : Engrailed as well as two other homeoproteins, Gbx2 and Lhx9, decrease the number of DSBs induced by H₂O₂

Given the high structural and functional conservation of homeoproteins, the Inventors have expected that other homeoproteins would have effects similar to that of Engrailed. They thus have compared effect on DNA damage induced by H₂O₂ of two other homeoproteins, Gbx2 (Lin et al., 1996, Genomics, 3, 335-342) and Lhx9 (Rétaux et al., 1999, J. of Neuroscience, 19(2), 783-793) compared to Engrailed.

E14.5 midbrain cells were dissociated by trypsin and cultured for 6 days in Neurobasal medium supplemented with glutamine, aspartate, glutamate and antibiotic/antimycotic mix on poly-D-lysine/laminin glass coverslips. Cells were then treated with the homeoprotein at the indicated concentration for 24h and then challenged for 1h with 100 uM H₂O₂ and fixed in 4% paraformaldehyde. DNA breaks were visualized with γ2HAX immunofluroescence. Each data point in the graphs represents the number of medium to large foci in the nucleus of a cell. 5-10 cells per coverslip and there were 4 coverslips per condition. Cells without foci were not included.

Differences between the groups were evaluated using the nonparametric Kruskal-Wallis followed by Dunn's Multiple Comparison Test.

### 7.1. Dose response for Engrailed 1 (figure 5.1)

Treatment with 100 µM H₂O₂ significantly increased the number of foci from 1.9 to 8.3/cell (p < 0.01). Pretreatment with En1 reduced the number of foci in a dose-dependent. 1 ng/ml (0.25 nM) partially but significantly (p < 0.01) reduced the number of foci caused by H2O2. 5 ng/ml (1.25 nM) and 20 ng/ml (0.5 nM) reduced the number of foci even further (p < 0.01) but were still greater than vehicle treated control condition (p < 0.01). 100 ng/ml (2.5 nM) was indistinguishable from control treated cells and thus completely prevented DNA breaks caused by H2O2.

### 7.2. Dose response for Gbx2 (Figure 5.2)

Treatment with H₂O₂ significantly doubled the number γ2HAX foci compared to vehicle treated control (p < 0.01). 1 ng/ml (0.027 nM) partially but significantly reduced the number of foci (p < 0.01). 20 ng/ml (0.54 nM) reduced somewhat further the number of foci and 100 ng/ml (2.68 nM) blocked the effects of H2O2.

***7.3. Dose response for Lhx9 (******Figure 5******.*** ***3***Treatment with H₂O₂ significantly doubled the number γ2HAX foci compared to vehicle treated control (p < 0.01). 5 ng/ml (0.022 nM) significantly reduced the number of foci and this reduction was even greater but not complete at 20 ng/ml (0.54 nM). 100 ng/ml (2.68 nM) completely blocked the effects of H2O2.

### Conclusion

Engrailed homeoproteins are expressed in adult dopaminergic neurons of the substantia nigra. In Engrailed1 heterozygous mice, these neurons start dying at 6 weeks, are more sensitive to oxidative stress, and progressively develop traits similar to those observed following an acute and strong oxidative stress inflected to wild-type neurons. These changes include DNA strand breaks and the modification (intensity and distribution) of several nuclear and nucleolar heterochromatin marks. Engrailed1 and Engrailed2 are biochemically equivalent transducing proteins previously used to antagonize dopaminergic neuron death in Engrailed1 heterozygous mice and in mouse models of Parkinson disease.

Accordingly, we show that, following an acute oxidative stress, a single Engrailed2 injection restores all nuclear and nucleolar heterochromatin marks, decreases the number of DNA strand breaks, and protects dopaminergic neurons against apoptosis. These results support the fact that Engrailed is not able only to prevent the damage of dopaminergic neurons present in dopaminergic neurons of the substantia nigra but also to rescue these neurons when damaged by oxidative stress.

High levels of reactive oxygen species (ROS) are toxic, in particular at the DNA level where they directly induce DDR. In consequence, neurons with high metabolic activity, such as SNpc mDA neurons, producing high amounts of ATP and ROS, are at risk for degeneration. Chromatin remodeling and DDR pathways are interconnected as DNA damage induces chromatin changes, themselves necessary to give access to the DNA repair machinery. This study places Engrailed genes as key regulators of DNA damage and chromatin changes that accompany chronic and acute forms of oxidative stress in mDA neurons.

The En+/- chronic model of oxidative stress in which mDA neurons show progressive but faster death rate than in WT mice demonstrates that, similar to Otx2 dosage in the adult retina, Engrailed dosage is important in the adult SNpc. Indeed, given that En1 and En2 are biochemically equivalent, the loss of only one allele out of four is enough to accelerate cell death. In the context of aging and neurological diseases, this suggests that mDA neurons in the En1+/- mouse age faster and are more sensitive to 6-OHDA, a toxin used in animal models of PD.

PD, even in its familial forms, manifests rather late in life, underscoring a risk associated with age. Even if Engrailed is not a PD gene, its anti-aging properties might explain the association of EN1 polymorphisms and the risk to develop PD. In this context, it is noteworthy that several phenotypes observed either in the En1+/- mice or in the acute 6-OHDA model are reminiscent of observations made in PD patients or models. For example, the loss of MeCP2 in SNpc mDA neurons compromises the nigrostriatal dopaminergic pathway; JNK and cyclin pathways are implied in PD and nucleolin diffusion was reported in PD patients.

In a previous study, it was shown that Engrailed protects mDA neurons in three mouse models of PD (Alvarez-Fischer *et al.,* 2011). The study was very different in the sense that 6-OHDA was injected in the striatum and not injected directly into the SNpc, an acute and harsh procedure preventing long-term secondary effects. More importantly, in the previous study, Engrailed was infused in the SNpc 3 weeks before the striatal insult and not injected 30 min after 6-OHDA injection. Finally, the present study, contrary to the previous ones, establishes a role of Engrailed as a transcriptional and epigenetic regulator and not only at the level of protein translation, even if the two modes of action may concur to save the cells. This transcriptional and epigenetic activity is very important as it suggests that Engrailed may have a long-lasting effect on mDA neuron survival. Further, the present study, contrary to the previous one, shows that Engrailed can be useful to rescue the dopamoinergic, not only to prevent their oxidative stress-induced injury.

One can thus propose a mechanism based on short-term and long-term effects. Short-term effects include the translation of mRNAs encoding mitochondrial proteins (Alvarez-Fischer et al., 2011: Stettler *et al.,* 2012) and, in particular but not only, the transcription of anti-apoptotic genes (this study) and the repression of apoptosis as already proposed (Alberi *et al.,* 2004; Beltran *et al.,* 2014), with a predominance, seemingly, of GADD45b/g, NF-kB, and JNK pathways. We propose that long-lasting effects relate to transcriptional and epigenetic mechanisms allowing both chromatin restructuration and DNA repair, two highly interconnected pathways based on the regulation of the genes highlighted in our initial RNA-seq study comparing the transcriptome of WT and En1+/- SNpc.

In conclusion, the protective effects of Engrailed in PD models (Alvarez-Fischer *et al.,* 2011), together with the present data, give credit to the idea of using Engrailed as a therapeutic protein acting at both the levels of mRNA translation and direct or indirect gene transcription.

## Claims

1. Engrailed protein for use in the treatment and/or prevention of DNA-damage in a patient suffering from Parkinson disease,
wherein said Engrailed protein is injected once into the substantia nigra pars compacta (SNpc) region.

2. Composition comprising Engrailed protein for use in the treatment and/or prevention of DNA-damage in a patient suffering from Parkinson disease, wherein said composition is injected once into the substantia nigra pars compacta (SNpc) region.

3. Composition for use according to claim 2, wherein said composition comprises another pharmaceutical drug selected in the group of growth factor, complex sugar and sirtuin.

4. Composition for use according to claim 3, wherein said complex sugar is polysialic acid.

## Patentansprüche

1. Engrailed-Protein zur Verwendung bei der Behandlung und/oder Vorbeugung von DNA-Schaden bei einem Patienten, der an der Parkinson-Krankheit leidet,
wobei das Engrailed-Protein einmal in die Substantia nigra pars compacta (SNpc)-Region injiziert wird.

2. Zusammensetzung, umfassend Engrailed-Protein zur Verwendung bei der Behandlung und/oder Vorbeugung von DNA-Schaden bei einem Patienten, der an der Parkinson-Krankheit leidet, wobei die Zusammensetzung einmal in die Substantia nigra pars compacta (SNpc)-Region injiziert wird.

3. Zusammensetzung zur Verwendung nach Anspruch 2, wobei die Zusammensetzung ein anderes pharmazeutisches Arzneimittel umfasst, das aus der Gruppe von Wachstumsfaktor, Komplexzucker und Sirtuin ausgewählt ist.

4. Zusammensetzung zur Verwendung nach Anspruch 3, wobei der Komplexzucker Polysialinsäure ist.

## Revendications

1. Protéine Engrailed pour son utilisation dans le traitement et/ou la prévention des dommages à l'ADN chez un patient souffrant de la maladie de Parkinson,
dans laquelle ladite protéine Engrailed est injectée une fois dans la région de la substantia nigra pars compacta (SNpc).

2. Composition comprenant une protéine Engrailed pour son utilisation dans le traitement et/ou la prévention des dommages à l'ADN chez un patient souffrant de la maladie de Parkinson, dans laquelle ladite composition est injectée une fois dans la région de la substantia nigra pars compacta (SNpc).

3. Composition pour son utilisation selon la revendication 2, dans laquelle ladite composition comprend un autre médicament pharmaceutique choisi parmi le groupe de facteur de croissance, sucre complexe et sirtuine.

4. Composition destinée à être utilisée selon la revendication 3, dans laquelle ledit sucre complexe est l'acide polysialique.
